# EUROPEAN PATENT SPECIFICATION

(11) **EP 1 533 380 B1**
(45) Date of publication and mention of the grant of the patent: **04.11.2009**
(21) Application number: 05100732.6
(22) Date of filing: 17.04.2000
(51) Int. Cl.: C12N 15/85, C07K 14/505, C12N 15/86, C12N 5/06

(54) **Recombinant protein production in a human cell comprising at least one E1 protein of adenovirus**
Verwendung von Rekombinanten Proteinen in Menschlichen Zellen
Production de protéine de recombinaison dans une cellule humaine comprenant au moins une protéine E1 d'adénovirus

(30) Priority: 15.04.1999 EP 99201176; 21.12.1999 EP 99204434
(43) Date of publication of application: 25.05.2005
(62) Divisional of application: 00921175.6
(73) Proprietor: Crucell Holland B.V., 2333 CN Leiden (NL)
(72) Inventor: Hateboer, Guus, 2105 SP Heemstede (NL); Verhulst, Karina Cornelia, 2497 XR Den Haag (NL); Schouten, Govert, 2353 EM Leiderdorp (NL); Uytdehaag Alphonsus, 3452 EH Vleuten (NL); Bout, Abraham, 2751 XL Moerkapelle (NL)
(74) Representative: Verhage, Richard Abraham

(56) References cited:
- EP-A- 0 185 573
- WO-A-93/03163
- WO-A-95/29994
- WO-A-97/00326
- FALLAUX, FRITS J. ET AL: "New helper cells and matched early region 1-deleted adenovirus vectors prevent generation of replication-competent adenoviruses." HUMAN GENE THERAPY, VOL. 9, NO. 13, PP. 1909-1917., 1998, XP002115715
- BOUTL, A. (1) ET AL: "A novel packaging cell line ( PER. C6 ) for efficient production of RCA-free batches of E1-deleted recombinant adenoviral vectors." CANCER GENE THERAPY, (NOV.-DEC., 1997) VOL. 4, NO. 6 CONF. SUPPL., PP. S32-S33. MEETING INFO.: SIXTH INTERNATIONAL CONFERENCE ON GENE THERAPY OF CANCER SAN DIEGO, CALIFORNIA, USA NOVEMBER 20-22, 1997, 1997, XP002115717
- BOUT, A. ET AL: "Production of RCA-free batches of E1-deleted recombinant adenoviral vectors on PER. C6" NUCLEIC ACIDS SYMP. SER. 38(ADVANCES IN GENE TECHNOLOGY: MOLECULA BIOLOGY IN THE CONQUEST OF DISEASE), 35-36, 1998, XP002115716 1998
- BOUT, A. (1) ET AL: "Improved helper cells for RCA-free production of E1-deleted recombinant adenovirus vectors." CANCER GENE THERAPY, VOL. 3, NO. 6 CONF. SUPPL., PP. S24. MEETING INFO.: FIFTH INTERNATIONAL CONFERENCE ON GENE THERAPY OF CANCER SAN DIEGO, CALIFORNIA, USA NOVEMBER 14-16, 1996, 1996, XP002115718
- ZHANG X ET AL: "Stable expression of human alpha-2,6-sialyltransferase in Chinese hamster ovary cells: functional consequences for human erythropoietin expression and bioactivity" BBA - GENERAL SUBJECTS, ELSEVIER SCIENCE PUBLISHERS, NL, vol. 1425, no. 3, 27 November 1998 (1998-11-27), pages 441-452, XP004276231 ISSN: 0304-4165
- GRABENHORST E ET AL: "CONSTRUCTION OF STABLE BHK-21 CELLS COEXPRESSING HUMAN SECRETORY GLYCOPROTEINS AND HUMAN GAL(BETA1-4)GLCNAC-R ALPHA2,6-SIALYLTRANSFERASE ALPHA2,6-LINKED NEUAC IS PREFERENTIALLY ATTACHED TO THE GAL(BETA1-4)GLCNAC(BETA1-2)MAN(ALPHA1-3)-B RANCH OF DIANTENNAR" EUROPEAN JOURNAL OF BIOCHEMISTRY, BERLIN, DE, vol. 232, no. 3, 15 September 1995 (1995-09-15), pages 718-725, XP001121694 ISSN: 0014-2956
- HOLLISTER J R ET AL: "STABLE EXPRESSION OF MAMMALIAN BETA1,4-GALACTOSYLTRANSFERASE EXTENDS THE N-GLYCOSYLATION PATHWAY IN INSECT CELLS" GLYCOBIOLOGY, IRL PRESS,, GB, vol. 8, no. 5, May 1998 (1998-05), pages 473-480, XP000981754 ISSN: 0959-6658
- MINCH SHERRILL L ET AL: "Tissue plasminogen activator coexpressed in Chinese hamster ovary cells with alpha(2,6)-sialyltransferase contains NeuAc-alpha(2,6)Gal-beta(1,4)Glc-N-AcR linkages" BIOTECHNOLOGY PROGRESS, vol. 11, no. 3, 1995, pages 348-351, XP002323097 ISSN: 8756-7938
- JENKINS N ET AL: "Getting the glycosylation right: implications for the biotechnology dustry" NATURE BIOTECHNOLOGY, NATURE PUBLISHING, US, vol. 14, no. 8, August 1996 (1996-08), pages 975-981, XP002133654 ISSN: 1087-0156
- WEIKERT S ET AL: "Engineering Chinese hamster ovary cells to maximize sialic acid content of recombinant glycoproteins" NATURE BIOTECHNOLOGY, NATURE PUB. CO, NEW YORK, NY, US, vol. 17, no. 11, November 1999 (1999-11), pages 1116-1121, XP002203703 ISSN: 1087-0156
- GRAND R J A ET AL: "MODULATION OF THE LEVEL OF EXPRESSION OF CELLULAR GENES IN ADENOVIRUS 12-INFECTED AND TRANSFORMED HUMAN CELLS." EMBO (EUR MOL BIOL ORGAN) J, 5 (6), 1253-1260., 1986, XP002115719
- GRAND, ROGER J. A. (1) ET AL: "The high levels of p53 present in adenovirus early region 1-transformed human cells do not cause up-regulation of MDM2 expression." VIROLOGY, VOL. 210, NO. 2, PP. 323-334., 1995, XP002115720
- YU, DIHUA ET AL: "Enhanced c-erbB-2/neu expression in human ovarian cancer cells correlates with more severe malignancy that can be suppressed by E1A" CANCER RES. 53(4), 891-8, 1993, XP002115721 1993

### SEQUENCE LISTING

<110> Crucell Holland B.V.
   Hateboer, Guus
   Verhulst, Karina C.
   Schouten, Govert J.
   Uytdehaag, Alphonsus G.C.M
   Bout, Abraham
<120> Recombinant protein reduction in a human cell
<130> 0034 EP 01 DIV
<140> PCT/NL00/00247
   <141> 2001-10-12
<160> 29
<170> PatentIn ver. 2.1
<210> 1
   <211> 41
   <212> DNA
   <213> Artificial sequence
<220>
   <223> Description of Artificial Sequence: primer DHFR up
<400> 1
   gatccacgtg agatctccac catggttggt tcgctaaact g 41
<210> 2
   <211> 37
   <212> DNA
   <213> Artificial Sequence
<220>
   <223> Description of Artificial Sequence: primer DHFR down
<400> 2
   gatccacgtg agatctttaa tcattcttct catatac 37
<210> 3
   <211> 85
   <212> DNA
   <213> Artificial sequence
<220>
   <223> Description of Artificial Sequence: polylinker fragment
<400> 3
   accggtgaat tcggcgcgcc gtcgacgata tcgatcggac cgacgcgttc gcgagcggcc 60
   gcaattcgct agcgttaacg gatcc 85
<210> 4
   <211> 86
   <212> DNA
   <213> Artificial Sequence
<220>
   <223> Description of Artificial Sequence: primer
<400> 4
   accggtgaat tgcggccgct cgcgaacgcg tcggtccgta tcgatatcgt cgacggcgcg 60
   ccgaattcgc tagcgttaac ggatcc 86
<210> 5
   <211> 43
   <212> DNA
   <213> Artificial Sequence
<220>
   <223> Description of Artificial sequence: oligonucleotide primer EPO-START
<400> 5
   aaaaaggatc cgccaccatg ggggtgcacg aatgtcctgc ctg 43
<210> 6
   <211> 38
   <212> DNA
   <213> Artificial Sequence
<220>
   <223> Description of Artificial sequence: oligonucleotide primer EPO-STOP
<400> 6
   aaaaaggatc ctcatctgtc ccctgtcctg caggcctc 38
<210> 7
   <211> 47
   <212> DNA
   <213> Artificial Sequence
<220>
   <223> Description of Artificial Sequence: primer LTR-1
<400> 7
   ctgtacgtac cagtgcactg gcctaggcat ggaaaaatac ataactg 47
<210> 8
   <211> 64
   <212> DNA
   <213> Artificial Sequence
<220>
   <223> Description of Artificial Sequence: primer LTR-2
<400> 8
   gcggatcctt cgaaccatgg taagcttggt accgctagcg ttaaccgggc gactcagtca 60
   atcg 64
<210> 9
   <211> 28
   <212> DNA
   <213> Artificial Sequence
<220>
   <223> Description of Artificial Sequence: primer HSA1
<400> 9
   gcgccaccat gggcagagcg atggtggc 28
<210> 10
   <211> 50
   <212> DNA
   <213> Artificial Sequence
<220>
   <223> Description of Artificial Sequence: primer HSA2
<400> 10
   gttagatcta agcttgtcga catcgatcta ctaacagtag agatgtagaa 50
<210> 11
   <211> 10
   <212> DNA
   <213> Artificial Sequence
<220>
   <223> Description of Artificial Sequence: oligonucleotide
<400> 11
   ttaagtcgac 9
<210> 12
   <211> 23
   <212> DNA
   <213> Artificial Sequence
<220>
   <223> Description of Artificial Sequence: oligo linker containing a PacI site
<400> 12
   aattgtctta attaaccgct taa 23
<210> 13
   <211> 67
   <212> DNA
   <213> Artificial Sequence
<220>
   <223> Description of Artificial Sequence: oligonucleotide primer PLL-1
<400> 13
   gccatcccta ggaagcttgg taccggtgaa ttcgctagcg ttaacggatc ctctagacga 60
   gatctgg 67
<210> 14
   <211> 67
   <212> DNA
   <213> Artificial Sequence
<220>
   <223> Description of Artificial Sequence: oligonucleotide primer PLL-2
<400> 14
   ccagatctcg tctagaggat ccgttaacgc tagcgaattc accggtacca agcttcctag 60
   ggatggc 67
<210> 15
   <211> 39
   <212> DNA
   <213> Artificial sequence
<220>
   <223> Description of Artificial Sequence: primer CMVplus
<400> 15
   gatcggtacc actgcagtgg tcaatattgg ccattagcc 39
<210> 16
   <211> 29
   <212> DNA
   <213> Artificial Sequence
<220>
   <223> Description of Artificial Sequence: primer CMVminA
<400> 16
   gatcaagctt ccaatgcacc gttcccggc 29
<210> 17
   <211> 34
   <212> DNA
   <213> Artificial Sequence
<220>
   <223> Description of Artificial Sequence: primer CAMH-UP
<400> 17
   gatcgatatc gctagcacca agggcccatc ggtc 34
<210> 18
   <211> 30
   <212> DNA
   <213> Artificial sequence
<220>
   <223> Description of Artificial Sequence: primer CAMH-DOWN
<400> 18
   gatcgtttaa actcatttac ccggagacag 30
<210> 19
   <211> 28
   <212> DNA
   <213> Artificial Sequence
<220>
   <223> Description of Artificial Sequence: primer CAML-UP
<400> 19
   gatccgtacg gtggctgcac catctgtc 28
<210> 20
   <211> 31
   <212> DNA
   <213> Artificial Sequence
<220>
   <223> Description of Artificial Sequence: primer CAML-DOWN
<400> 20
   gatcgtttaa acctaacact ctcccctgtt g 31
<210> 21
   <211> 20
   <212> PRT
   <213> Artificial sequence
<220>
   <223> Description of Artificial Sequence: leader sequence
<400> 21
   Met Ala Cys Pro Gly Phe Leu Trp Ala Leu Val Ile Ser Thr Cys Leu
    1               5                  10                  15
   Glu Phe Ser Met
               20
<210> 22
   <211> 60
   <212> DNA
   <213> Artificial sequence
<220>
   <223> Description of Artificial Sequence: leader sequence
<400> 22
   atggcatgcc ctggcttcct gtgggcactt gtgatctcca cctgtcttga attttccatg 60
<210> 23
   <211> 38
   <212> DNA
   <213> Artificial Sequence
<220>
   <223> Description of Artificial Sequence: primer UBS-UP
<400> 23
   gatcacgcgt gctagccacc atggcatgcc ctggcttc 38
<210> 24
   <211> 28
   <212> DNA
   <213> Artificial Sequence
<220>
   <223> Description of Artificial Sequence: primer UBSHV-DOWN
<400> 24
   gatcgctagc tgtcgagacg gtgaccag 28
<210> 25
   <211> 29
   <212> DNA
   <213> Artificial Sequence
<220>
   <223> Description of Artificial Sequence: primer UBSLV-DOWN
<400> 25
   gatccgtacg cttgatctcc accttggtc 29
<210> 26
   <211> 50
   <212> DNA
   <213> Artificial Sequence
<220>
   <223> Description of Artificial Sequence: primer 15C5-UP
<400> 26
   gatcacgcgt gctagccacc atgggtactc ctgctcagtt tcttggaatc 50
<210> 27
   <211> 41
   <212> DNA
   <213> Artificial Sequence
<220>
   <223> Description of Artificial Sequence: forward primer
<400> 27
   attggcgcgc caccatgaag actatcattg ctttgagcta c 41
<210> 28
   <211> 39
   <212> DNA
   <213> Artificial Sequence
<220>
   <223> Description of Artificial Sequence: reverse primer
<400> 28
   gatgctagct catctagttt gtttttctgg tatattccg 39
<210> 29
   <211> 42
   <212> DNA
   <213> Artificial Sequence
<220>
   <223> Description of Artificial Sequence: another reverse primer
<400> 29
   gatgctagct cagtctttgt atcctgactt cagttcaaca cc 42

## Description

The invention relates to the embodiments characterized in the claims. Thus, it relates to the field of recombinant protein production, more in particular to the use of a human cell for the production of proteins. The invention further relates to the field of monoclonal antibodies production, and more in particular to the use of a human cell for the production of monoclonal antibodies. The invention further relates to the field of production of viral proteins. The invention is particularly useful for the production of vaccines to aid in protection against viral pathogens for vertebrates, in particular mammalians and especially humans.

Methods and compositions are disclosed herein for the production of recombinant proteins. The invention is particularly useful for the production of proteins that require post-translational or peritranslational modifications such as glycosylation and proper folding. The expression of human recombinant proteins in heterologous cells has been well documented. Many production systems for recombinant proteins have become available, ranging from bacteria, yeasts, and fungi to insect cells, plant cells and mammalian cells. However, despite these developments, some production systems are still not optimal, or only suited for production of specific classes of proteins. For instance, proteins that require post- or peri-translational modifications such as glycosylation,
γ-carboxylation, or γ-hydroxylation can not be produced in prokaryotic production systems. Another well-known problem with prokaryotic expression systems is the often incorrect folding of the product to be produced, even leading to insoluble inclusion bodies in many cases. Eukaryotic systems are an improvement in the production of, in particular eukaryote derived proteins, but the available production systems still suffer from a number of drawbacks. The hypermannosylation in for instance yeast strains affect the ability of yeasts to properly express glycoproteins. Hypermannosylation often even leads to immune reactions when a therapeutic protein thus prepared is administered to a patient. Furthermore, yeast secretion signals are different from mammalian signals leading to a more problematic transport of mammalian proteins, including human polypeptides, to the extracellular, which in turn results in problems with continuous production and/or isolation. Mammalian cells are widely used for the production of such proteins because of their ability to perform extensive post-translational modifications. The expression of recombinant proteins in mammalian cells has evolved dramatically over the past years, resulting in many cases in a routine technology.
In particular, Chinese Hamster Ovary cells (CHO) have become a routine and convenient production system for the generation of biopharmaceutical proteins and proteins for diagnostic purposes. A number of characteristics make CHO very suitable as a host cell: the production levels that can be reached in CHO cells are extremely high; the cell line provides a safe production system, which is free of infectious or virus-like particles; CHO cells have been characterized extensively, although the history of the original cell line is vague; CHO cells can grow in suspension till high densities in bioreactors, using serum-free culture media; a dhfr- mutant of CHO (DG-44 clone.Urlaub et al, 1983) has been developed to obtain an easy selection system by introducing an exogenous dhfr gene and thereafter a well controlled amplification of the dhfr gene and the transgene using methotrexate.
However, glycoproteins or proteins comprising at least two (different) subunits continue to pose problems. The biological activity of glycosylated proteins can be profoundly influenced by the exact nature of the oligosaccharide component. The type of glycosylation can also have significant effects on immunogenicity, targeting and pharmacokinetics of the glycoprotein. In recent years, major advances have been made in the cellular factors that determine the glycosylation, and many glycosyl transferase enzymes have been cloned. This has resulted in research aimed at metabolic engineering of the glycosylation machinery (Fussenegger et al, 1999; Lee et al, 1989; Vonach et al, 1998; Jenkins et al, 1998; Zhang et al, 1998; Muchmore et al, 1989). Examples of such strategies are described below.
CHO cells lack a functional a-2,6 sialyl-transferase enzyme, resulting in the exclusive addition of sialic acids to galactose via a-2,3 linkages. It is known that the absence of a-2,6 linkages can enhance the clearance of a protein form the bloodstream. To address this problem, CHO cells have been engineered to resemble the human glycan profile, by transfection of the appropriate glycosyl transferases. CHO cells are also incapable of producing Lewis^{X} oligosaccharides. CHO cell lines have been developed that express human N-acetyl-D-glucosaminyltransferase and a-1,3-fucosyl-transferase III. In contrast, it is known that rodent cells, including CHO cells, produce CMP-N-acetylneuraminic acid hydrolase which glycosylates CMP-N-acetylneuraminic acids (Jenkins et al, 1996), an enzyme that is absent in humans. The proteins that carry this type of glycosylation can produce a strong immune response when injected (Kawashima et al, 1993). The recent identification of the rodent gene that encodes the hydrolase enzyme will most likely facilitate the development of CHO cells that lack this activity and will avoid this rodent type modification.
The art thus teaches that it is possible to alter the glycosylation potential of mammalian host cells by expression of human glycosyl transferase enzymes. Yet, although the CHO-derived glycan structures on the recombinant proteins may mimic those present on their natural human counterparts, they are still found to be far from identical. Another potential problem is that not all glycosylation enzymes have been cloned and are therefore available for metabolic engineering. The therapeutic administration of proteins that differ from their natural human counterparts may result in activation of the immune system of the patient and cause undesirable responses that may affect the efficacy of the treatment. Other problems using non-human cells may arise from incorrect folding of proteins that occurs during or after translation which might be dependent on the presence of the different available chaperone proteins. Aberrant folding may occur, leading to a decrease or absence of biological activity of the protein. Furthermore, the simultaneous expression of separate polypeptides that will together form proteins comprised of the different subunits, like monoclonal antibodies, in correct relative abundancies is of great importance. Human cells will be better capable of providing all necessary facilities for human proteins to be expressed and processed correctly.
It is thus clearly desirable to have methods for producing human recombinant proteins that involve a human cell that provides consistent human type processing like post-translational and peritranslational modifications, such as glycosylation, which preferably is also suitable for large scale production.

Thus the invention provides a method for producing at least one proteinaceous substance in a PER.C6 cell, which cell does not encode a structural adenoviral protein from its genome or a sequence integrated therein, said method comprising providing said cell with a gene encoding a recombinant proteinaceous substance, culturing said cell in a suitable medium and harvesting at least one proteinaceous substance from said cell and/or said medium. A proteinaceous substance is a substance comprising at least two aminoacids linked by a peptide bond. The substance may further comprise on or more other molecules physically linked to said amino acid portion or not. Non-limiting examples of such other molecules include carbohydrate and/or lipid molecules. Nucleic acid encoding an adenovirus structural protein should not be present for a number of reasons. One of the reasons is that the presence of an adenovirus structural protein in a preparation of produced protein, is highly undesired in many applications of such produced protein. Removal of such structural protein from the product is best achieved by avoiding its occurrence in the preparation. In a preferred embodiment, the proteinaceous substance harvested from the cell and the cell itself are derived from the same species. For instance if the protein is intended to be administered to humans it is preferred that both the cell and the proteinaceous substance harvested from said cell are of human origin. One advantage of a human cell is that most of the commercially most attractive proteins are human. The proteinaceous substance harvested from said cell can be any proteinaceous substance produced by said cell. In one embodiment at least one of said at least one harvested proteinaceous substance is encoded by said gene. In another embodiment, a gene is provided to said cell to enhance and/or induce expression of one ore more endogenously present genes in a cell. For instance by providing said cell with a gene encoding a protein that is capable of enhancing expression of a proteinaceous substance in said cell.
With a gene is meant a nucleic acid comprising a nucleic acid of interest in an expressible format, such as an expression cassette. The nucleic acid of interest may be expressed form the natural promoter or derivative thereof or an entirely heterologous promoter. The nucleic acid of interest can comprise introns or not. Similarly, it may be a cDNA or cDNA-like nucleic acid. The nucleic acid of interest may encode a protein. Alternatively, the nucleic acid of interest can encode an anti-sense RNA.

The invention further provides a method for producing at least one human recombinant protein in a cell, comprising providing a human PER.C6 cell which cell does not produce structural adenoviral proteins, with a nucleic acid encoding said human recombinant protein, culturing said cell in a suitable medium and harvesting at least one human recombinant protein from said cell and/or said medium. Until the present invention there are few, if any human cells that have been found suitable to produce human recombinant proteins in any reproducible and upscaleable manner. We have now found that PER.C6 cells, which comprise at least immortalising adenoviral E1 sequences in their genome, are capable of growing (they are immortalized by the presence of E1), relatively independent of exogenous growth factors. Furthermore, these cells are capable of producing recombinant proteins in significant amounts and which are capable of correctly processing the recombinant protein being made. Of course these cells will also be capable of producing non-human proteins. The human cell lines that have been used to produce recombinant proteins in any significant amount are often tumor (transformed) cell lines. The fact that most human cells that have been used for recombinant protein production are tumor-derived adds an extra risk to working with these particular cell lines and results in very stringent isolation procedures for the recombinant protein in order to avoid transforming activity or tumorigenic material in any protein or other preparations. In the method according to the invention said cell is a PER.C6 cell, which is derived from a primary human embryonic retinoblast (HER) cell. In order to be able to grow indefinitely, a primary cell needs to be immortalized in some kind, which in the present invention has been achieved by the introduction of Adenovirus E 1. The art is not clear on what the border is between transformed and immortalized. In here the difference is meant to represent that immortalized cells grow indefinitely, while the phenotype is still present and transformed cells also grow indefinitely but also display usually a dramatic change in phenotype. In order to achieve large scale (continuous) production of recombinant proteins through cell culture it is preferred in the art to have cells capable of growing without the necessity of anchorage. The cells of the present invention have that capability. The anchorage-independent growth capability is improved when the cells comprise a sequence encoding E2A or a functional derivative or analogue or fragment thereof in its genome, whereby preferably said E2A encoding sequence encodes a temperature sensitive mutant E2A, such as ts125. To have a clean and safe production system from which it is easy to isolate the desired recombinant protein it is preferred to have a method according to the invention, whereby said human cell comprises no other adenoviral sequences. The cell for the methods and uses of the invention is PER.C6 as deposited under ECACC no. 96022940 or a derivative thereof. PER.C6 behaves better in handling than for instance transformed human 293 cells that have also been immortalized by the E1 region from Adenovirus. PER.C6 cells have been characterized and have been documented very extensively, while they behave significantly better in the process of upscaling, suspension growth and growth factor independence. Especially the fact that PER.C6 can be brought in suspension in a highly reproducible manner is something that makes it very suitable for large scale production. Furthermore, the PER.C6 cell line has been characterized for bioreactor growth in which it grows to very high densities.
The PER.C6 cells according to the invention have the additional advantage that they can be cultured in the absence of animal- or human-derived serum or animal- or human-derived serum components. Thus isolation is easier, while the safety is enhanced due to the absence of additional human or animal proteins in the culture and the system is very reliable (synthetic media are the best in reproducibility). Furthermore, the presence of the Early region 1A (E1A) of Adenovirus adds another level of advantages as compared to (human) cell lines that lack this particular gene: E1A as a transcriptional activator is known to enhance transcription from the enhancer/promoter of the Cytomegalovirus Immediate Early genes (Olive et al, 1990; Gorman et al, 1989). When the recombinant protein to be produced is under the control of the CMV enhancer/promoter expression levels increase in said cells and not in cells that lack E1A. Like the CMV promoter also E1A promoters are more active in cells expressing one or more E1A products, than in cells not expressing such products. It is known that indeed the E1A expression enhancement is a characteristic of several other promoters. For the present invention, such promoters are considered to be functional homologues of E1A promoters. The E1A effect can be mediated through the attraction of transcription activators the E1A promoter or homologue thereof and/or through the removal/avoiding attachment of transcriptional repressors to the promoter. The binding of activators and repressors to a promoter occurs in a sequence dependent fashion. A functional derivative and or fragment of an E1A promoter or homologue thereof, therefor at least comprises the nucleic acid binding sequence of at least one E1A protein regulated activator and/or repressor.
Another advantage of cells of the invention is that they harbor and express constitutively the Adenovirus E1B gene. Adenovirus E1B is a well-known inhibitor of programmed cell death, or apoptosis. This inhibition occurs either through the 55K E1B product by its binding to the transcription factor p53 and subsequent inhibition (Yew and Berk, 1992). The other product of the E1B region, 19K E1B, can prevent apoptosis by binding and thereby inhibiting the cellular death proteins Bax and Bak, both proteins that are under the control of p53 (White et al, 1992; Debbas and White, 1993; Han et al, 1996; Farrow et al. 1995). These features can be extremely useful for the expression of recombinant proteins that, when overexpressed, might be involved in the induction of apoptosis through a p53-dependent pathway.

The invention further provides the use of a human PER.C6 cell for the production of a human recombinant protein, which cell does not produce structural adenoviral proteins.
The invention further provides a use according to the invention, wherein said cell further comprises a sequence encoding E2A or a functional derivative or analogue or fragment thereof in its genome, preferably wherein said E2A is temperature sensitive.
The invention also provides a human recombinant protein obtainable by a method according to the invention or by a use according to the invention, said human recombinant protein having a human glycosylation pattern different from the isolated natural human counterpart protein.
In another embodiment the invention provides a human cell, which cell does not produce structural adenoviral proteins and having a gene encoding a human recombinant protein, which is a cell which is derived from PER.C6 as deposited under ECACC no. 96022940. In yet another embodiment the invention provides such a human cell, PER.C6/E2A, which further comprises a sequence encoding E2A or a functional derivative or analogue or fragment thereof in its genome, preferably wherein said E2A is temperature sensitive. Proteins to be expressed in these cells and using the methods of the invention are well known to persons skilled in the art. They are preferably human proteins that preferably undergo some kind of processing in nature, such as secretion, chaperoned folding and/or transport, cosynthesis with other subunits, glycosylation, phosphorylation. Typical examples for therapeutic or diagnostic use include monoclonal antibodies that are comprised of several subunits, tissue specific Plasminogen Activator (tPA), granulocyte colony stimulating factor (G-CSF) and human erythropoietin (EPO). EPO is a typical product that, especially in vivo, heavily depends on its glycosylation pattern for its activity and immunogenicity. Thus far, relatively high levels of EPO can be reached by the use of CHO cells which is differently glycosylated when compared to EPO purified from human urine, albeit equally active in the enhancement of erythrocytes production. The different glycosylation of such EPO, however, leads to immunogenicity problems and altered half life problems in a recipient.

The present invention also discloses a novel human immortalized cell line for this purpose, and the uses thereof for production. PER.C6 cells (WO 97/00326) were generated by transfection of primary human embryonic retina cells, using a plasmid that contained the Adenovirus serotype 5 (Ad5) E1A- and E1B-coding sequences (Ad5 nucleotides 459-3510) under the control of the human phosphoglycerate kinase (PGK) promoter.
The following features make PER.C6 particularly useful as a host for recombinant protein production: 1. fully characterized human cell line; 2. developed in compliance with GLP; 3. can be grown as suspension cultures in defined serum-free medium devoid of any human- or animal-derived proteins; 4. growth compatible with roller bottles, shaker flasks, spinner flasks and bioreactors with doubling times of about 35 hrs; 5. presence of E1A causing an upregulation of expression of genes that are under the control of the CMV enhancer/promoter; 6. presence of E1B which prevents p53-dependent apoptosis possibly enhanced through overexpression of the recombinant transgene.
In one embodiment the invention provides a method of the invention wherein said cell is capable of producing 2 to 200-fold more recombinant protein and/or proteinaceous substance than conventional mammalian cell lines. Preferably, said conventional mammalian cell lines are selected from the group consisting of CHO, COS, Vero, Hela, BHK and Sp-2 cell lines.

In one aspect of the invention the proteinaceous substance or protein is a monoclonal antibody. Antibodies, or immunoglobulins (Igs), are serum proteins that play a central role in the humoral immune response, binding antigens and inactivating them or triggering the inflammatory response which results in their elimination. Antibodies are capable of highly specific interactions with a wide variety of ligands, including tumor-associated markers, viral coat proteins, and lymphocyte cell surface glycoproteins. They are, therefore, potentially very useful agents for the diagnosis and treatment of human diseases. Recombinant monoclonal and single chain antibody technology is opening new perspectives for the development of novel therapeutic and diagnostic agents. Mouse monoclonal antibodies have been used as therapeutic agents in a wide variety of clinical trials to treat infectious diseases and cancer. The first report of a patient being treated with a murine monoclonal antibody was published in 1980 (Nadler et al. 1980). However, the effects observed with these agents have in general been quite disappointing (for reviews see Lowder et al. 1986, Mellstedt et al. 1991, Baldwin and Byers 1986). Traditionally, recombinant monoclonal antibodies (immunoglobulins) are produced on B-cell hybridomas. Such hybridomas are produced by fusing an immunoglobulin-producing B-cell, initially selected for its specificity, to a mouse myeloma cell and thereby immortalizing the B-cell. The original strategy of immortalizing mouse B-cells was developed in 1975 (Köhler and Milstein). However, immunoglobulins produced in such hybridomas have the disadvantage that they are of mouse origin, resulting in poor antibody specificity, low antibody affinity and a severe host anti-mouse antibody response (HAMA, Shawler et al. 1985). This HAMA response may lead to inflammation, fever, and even death of the patient.
Mouse antibodies have a low affinity in humans and for reasons yet unknown have an extremely short half-life in human circulation (19-42 hours) as compared to human antibodies (21 days, Frödin et al. 1990). That, together with the severity of the HAMA response, has prompted the development of alternative strategies for generating more human or completely humanized immunoglobulins (reviewed by Owens and Young 1994, Sandhu 1992, Vaswani et al. 1998).
One such strategy makes use of the constant regions of the human immunoglobulin to replace its murine counterparts, resulting in a new generation of "chimeric" and "humanized" antibodies. This approach is taken since the HAMA response is mainly due to the constant domains (Oi et al, 1983; Morrison et al, 1984). An example of such a chimeric antibody is CAMPATH-1H (Reichmann et al. 1988). The CAMPATH-1H Ab, used in the treatment of non-Hodgkin's B-cell lymphoma and refractory rheumatoid arthritis, is directed against the human antigen CAMPATH-1 (CDw52) present on all lymphoid cells and monocytes but not on other cell types (Hale et al. 1988, Isaacs et al. 1992). Other examples are Rituxan (Rituximab) directed against human CD20 (Reff et al. 1994) and 15C5, a chimeric antibody raised against human fragment-D dimer (Vandamme et al. 1990, Bulens et al. 1991) used in imaging of blood clotting. However, since these new generation chimeric antibodies are still partly murine, they can induce an immune response in humans, albeit not as severe as the HAMA response against fully antibodies of mouse origin. In another, more sophisticated approach, ranges of residues present in the variable domains of the antibody, but apparently not essential for antigen recognition, are replaced by more human-like stretches of amino acids, resulting in a second generation or hyperchimeric antibodies (Vaswani et al. 1998). A well known example of this approach is Herceptin (Carter et a1. 1992), an antibody that is 95% human, which is directed against HER2 (a tumor-specific antigen) and used in breast tumor patients.
A more preferred manner to replace mouse recombinant immunoglobulins would be one resulting in the generation of human immunoglobulins. Importantly, since it is unethical to immunize humans with experimental biological materials, it is not feasible to subsequently select specific B-cells for immortalization as was shown for mouse B-cells (Köhler and Milstein 1975). Although B-cells from patients were selected for specific antibodies against cancer antigens, it is technically more difficult to prepare human immunoglobulins from human material as compared to mouse antibodies (Köhler and Milstein, 1975). A recombinant approach to produce fully human antibodies became feasible with the use of phage displayed antibody libraries, expressing variable domains of human origin (McCafferty et al. 1990, Clarkson et al. 1991, Barbas et al. 1991, Garrard et al. 1991, Winter et al. 1994, Burton and Barbas, 1994). These variable regions are selected for their specific affinity for certain antigens and are subsequently linked to the constant domains of human immunoglobulins, resulting in human recombinant immunoglobulins. An example of this latter approach is the single chain Fv antibody 17-1A (Riethmuller et al. 1994) that was converted into an intact human IgG1 kappa immunoglobulin named UBS-54, directed against the tumor-associated EpCAM molecule (Huls et al. 1999).
The production systems to generate recombinant immunoglobulins are diverse. The mouse immunoglobulins first used in clinical trials were produced in large quantities in their parental-specific B-cell and fused to a mouse myeloma cell for immortalization. A disadvantage of this system is that the immunoglobulins produced are entirely of mouse origin and render a dramatic immune response (HAMA response) in the human patient (as described above).
Partially humanized or human antibodies lack a parental B-cell that can be immortalized and therefore have to be produced in other systems like Chinese Hamster Ovary (CHO) cells or Baby Hamster Kidney (BHK) cells. It is also possible to use cells that are normally suited for immunoglobulin production like tumor-derived human- or mouse myeloma cells. However, antibody yields obtained in myeloma cells are in general relatively low (±0.1 ug/ml) when compared to those obtained in the originally identified and immortalized B-cells that produce fully murine immunoglobulins (±10 ug/ml, Sandhu 1992). To circumvent these and other shortcomings, different systems are being developed to produce humanized or human immunoglobulins with higher yields.
For example, it was recently shown that transgenic mouse strains can be produced that have the mouse IgG genes replaced with their human counterparts (Bruggeman et al., 1991, Lonberg et al., 1994, Lonberg and Huszar, 1995, Jacobovits, 1995). Yeast artificial chromosomes (YACs) containing large fragments of the human heavy and light (kappa) chain immunoglobulin (Ig) loci were introduced into Ig-inactivated mice, resulting in human antibody production which closely resembled that seen in humans, including gene rearrangement, assembly, and repertoire (Mendez et al 1997, Green et al. 1994). Likewise, Fishwild *et al.* (1996) have constructed human Ig-transgenics in order to obtain human immunoglobulins using subsequent conventional hybridoma technology. The hybridoma cells secreted human immunoglobulins with properties similar to those of wild-type mice including stability, growth, and secretion levels. Recombinant antibodies produced from such transgenic mice strains carry no non-human amino acid sequences.

Nevertheless, human immunoglobulins produced thus far have the disadvantage of being produced in non-human cells, resulting in non-human post-translational modifications like glycosylation and/or folding of the subunits. All antibodies are glycosylated at conserved positions in their constant regions, and the presence of carbohydrates can be critical for antigen clearance functions such as complement activation. The structure of the attached carbohydrate can also affect antibody activity. Antibody glycosylation can be influenced by the cell in which it is produced, the conformation of the antibody and cell culture conditions. For instance, antibodies produced in mouse cells carry glycans containing the Gal alpha 1-3Gal residue, which is absent in proteins produced in human cells (Borrebaeck et al. 1993, Borrebaeck. 1999). A very high titer of anti-Gal alpha 1-3Gal antibodies is present in humans (100 ug/ml, Galili, 1993) causing a rapid clearance of (murine) proteins carrying this residue in their glycans.
It soon became apparent that in order to exert an effect, patients need to be treated with very high doses of recombinant immunoglobulins for prolonged periods of time. It seems likely that post-translational modifications on human or humanized immunoglobulins that are not produced on human cells strongly affect the clearance rate of these antibodies from the bloodstream.
It is unclear why immunoglobulins produced on CHO cells also need to be applied in very high dosages, since the Gal alpha1-3Gal residue is not present in glycans on proteins derived from this cell line (Rother and Squinto. 1996). Therefore, other post-translational modifications besides the Gal alpha1-3Gal residues are likely to be involved in specific immune responses in humans against fully human or humanized immunoglobulins produced on such CHO cells.

The art thus teaches that it is possible to produce humanized antibodies without murine-derived protein sequences. However, the current generation of recombinant immunoglobulins still differs from their natural human counterparts, e.g. by post-translational modifications such as glycosylation and folding. This may result in activation of the immune system of the patient and cause undesirable responses that may affect the efficacy of the treatment. Thus, despite the development of chimeric antibodies, the current production systems still need optimization to produce fully human or humanized active antibodies.
It is thus clearly desirable to have methods for producing fully human antibodies which behave accordingly, and which are, in addition, produced at higher yields than observed in human myeloma cells.

Thus, it would be an improvement in the art to provide a human cell that produces consistent human type protein processing like post-translational and peri-translational modifications, such as, but not limited to, for instance glycosylation. It would be further advantageous to provide a method for producing a recombinant mammalian cell and immunoglobulins from recombinant mammalian cells in large-scale production.

The present invention therefore further provides a method for producing at least one variable domain of an immunoglobulin in a recombinant mammalian cell, comprising providing a PER.C6 cell further comprising a nucleic acid encoding said immunoglobulin, culturing said cell in a suitable medium and harvesting at least one monoclonal antibody from said cell and/or said medium.
Previously, few, if any, human cells suitable for producing immunoglobulins in any reproducible and upscaleable manner have been found. The cells of the present invention comprise at least an immortalizing adenoviral E1 protein and are capable of growing relatively independent of exogenous growth factors.
Furthermore, these cells are capable of producing immunoglobulins in significant amounts and are capable of correctly processing the generated immunoglobulins.
The fact that cell types that have been used for immunoglobulin production are tumor-derived adds an extra risk to working with these particular cell lines and results in very stringent isolation procedures for the immunoglobulins in order to avoid transforming activity or tumorigenic material in any preparations. It is therefore preferred to employ a method according to the invention, whereby said cell is derived from a primary cell. In order to be able to grow indefinitely, a primary cell needs to be immortalized, which in the present invention has been achieved by the introduction of an adenoviral E1 protein.
In order to achieve large-scale (continuous) production of immunoglobulins through cell culture, it is preferred to have cells capable of growing without the necessity of anchorage. The cells of the present invention have that capability. The anchorage-independent growth capability is improved when the cells comprise an adenovirus-derived sequence encoding E2A (or a functional derivative or analogue or fragment thereof) in its genome. In a preferred embodiment the E2A encoding sequence encodes a temperature sensitive mutant E2A, such as ts125. Said cell may, in addition, comprise a nucleic acid (e.g. encoding tTa), which allows for regulated expression of a gene of interest when placed under the control of a promoter (e.g., a TetO promoter).
The nucleic acid may encode a heavy chain, a variable heavy chain, a light chain, and/or a variable light chain of an immunoglobulin. Alternatively, a separate or distinct nucleic acid may encode one or more variable domain(s) of an Ig (or a functional derivative, homologue and/or fragment thereof), as a counterpart to the first nucleic acid (described above). One or more nucleic acid(s) described herein may encode an ScFv and may be human or humanized. The nucleid acid(s) of the present invention are preferably placed under the control of an inducible promoter (or a functional derivative thereof).
To have a clean and safe production system from which it is easy to isolate the desired immunoglobulins, it is preferred to have a method according to the invention, whereby said human cell includes no other adenoviral sequences. The cell for the methods and uses of the invention is PER.C6 or a derivative thereof as deposited under ECACC no. 96022940. PER.C6 has been found to be more stable, particularly in handling, than, for instance, transformed human 293 cells immortalized by the adenoviral E1 region. PER.C6 cells have been extensively characterized and documented, demonstrating good process of upscaling, suspension growth and growth factor independence. Furthermore, PER.C6 can be incorporated into a suspension in a highly reproducible manner, making it particularly suitable for large-scale production. In this regard, the PER.C6 cell line has been characterized for bioreactor growth, where it can grow to very high densities.
The cells of the present invention, in particular PER.C6, can advantageously be cultured in the absence of animal- or human-derived serum, or animal- or human-derived serum components. Thus, isolation of monoclonal antibodies is simplified and safety is enhanced due to the absence of additional human or animal proteins in the culture. The absence of serum further increases reliability of the system since use of synthetic media as contemplated herein, enhances reproducibility. The invention further provides the use of a recombinant PER.C6 cell for the production of at least one variable domain of an immunoglobulin, which cell does not produce structural adenoviral proteins.
The invention further provides a use according to the invention, wherein said cell further comprises a sequence encoding E2A (or a functional derivative or analogue or fragment thereof) in its genome, preferably wherein said E2A is temperature sensitive. In addition the invention provides a method of using the invention, wherein said cell further comprises a trans-activating protein for the induction of said inducible promoter. The invention also provides immunoglobulins obtainable by a method according to the invention or by a use according to the invention.
Immunoglobulins to be expressed in the cells of the present invention are known to persons skilled in the art. Examples of recombinant immunoglobulins include, but are not limited to, Herceptin, Rituxan (Rituximab), UBS-54, CAMPATH-1H and 15C5.
The present invention further provides methods for producing at least one variable domain of an immunoglobulin in a recombinant PER.C6 cell utilizing the immortalized recombinant PER.C6 cell of the invention, culturing the same in a suitable medium, and harvesting at least one variable domain of a selected Ig from the recombinant PER.C6 cell and/or medium. Immunoglobulins, variable domains of the immunoglobulins, or derivatives thereof, may be used for the therapeutic treatment of mammals or the manufacture of pharmaceutical compositions.

In another aspect the invention provides a method for producing a viral protein other than adenovirus or adenoviral protein for use as a vaccine comprising providing a PER.C6 cell with a nucleic acid encoding said viral protein, culturing said cell in a suitable medium allowing for expression of said viral protein and harvesting viral protein from said medium and/or said cell. Until the present invention there are few, if any (human) cells that have been found suitable to produce viral proteins for use as vaccines in any reproducible and upscaleable manner and/or sufficiently high yields and/or easily purifiable. We have now found that PER.C6 cells, which comprise adenoviral E1 sequences in their genome, are capable of producing the viral protein in significant amounts.
The cell according to the invention is derived from a human primary HER cell which is immortalised by a gene product of said E1 gene. In order to be able to grow a primary cell of course needs to be immortalized.
A good example of such a cell is one derived from a human embryonic retinoblast.
In cells according to the invention it is important that the E1 gene sequences are not lost during the cell cycle. Said sequence encoding at least one gene product of the E 1 gene is present in the genome of said (human) PER.C6 cell. For reasons of safety care is best taken to avoid unnecessary adenoviral sequences in the cells according to the invention. It is thus another embodiment of the invention to provide cells that do not produce adenoviral structural proteins. However, in order to achieve large scale (continuous) virus protein production through cell culture it is preferred to have cells capable of growing without needing anchorage. The cells of the present invention have that capability. To have a clean and safe production system from which it is easy to recover and, if desirable, to purify the virus protein, it is preferred to have a method according to the invention, whereby said human cell comprises no other adenoviral sequences. The cell for the methods and uses of the invention is PER.C6 as deposited under ECACC no. 96022940, or a derivative thereof. Thus the invention provides a method using a cell according to the invention, wherein said cell further comprises a sequence encoding E2A or a functional derivative or analogue or fragment thereof, preferably a cell wherein said sequence encoding E2A or a functional derivative or analogue or fragment thereof is present in the genome of said human cell and most preferably a cell wherein said E2A encoding sequence encodes a temperature sensitive mutant E2A.
Furthermore, as stated the invention also provides a method according to the invention wherein said (human) cell is capable of growing in suspension.
The invention also provides a method wherein said human cell can be cultured in the absence of serum. The cells according to the invention, in particular PER.C6 has the additional advantage that it can be cultured in the absence of serum or serum components. Thus isolation is easy, safety is enhanced and reliability of the system is good (synthetic media are the best in reproducibility). The human PER.C6 cells of the invention are capable of normal post and peritranslational modifications and assembly. This means that they are very suitable for preparing viral proteins for use in vaccines.
Thus the invention provides a method according to the invention, wherein said viral protein comprises a protein that undergoes post-translational and/or peritranslational modification, especially wherein said modifications comprise glycosylation. The vaccine can be used for the vaccination of humans. However, the vaccine is also effective in animals. In this embodiment the vaccine is preferably produced accoridng to the invention wherein said cell is derived from the same species as the species the viral protein in the vaccine is derived from. A viral protein may be the entire viral protein or a functional equivalent thereof. A functional equivalent of a viral protein is at least an immunogenic part of the viral protein. A good example of a viral vaccine that has been cumbersome to produce in any reliable manner is influenza vaccine. The invention provides a method according the invention wherein said viral proteins comprise at least one of an Influenza virus neuraminidase and/or a haemagglutinin. Other viral proteins (subunits) that can be produced in the methods according to the invention include proteins from enterovirus, such as rhinovirus, aphtovirus, or poliomyelitisvirus, herpesvirus, such as herpes symplex virus, pseudorabies virus or bovine herpes virus, orthomyxovirus, such as influenza virus, a paramyxovirus, such as newcastle disease virus, respiratory syncitio virus, mumps virus or a measles virus, retrovirus, such as human immunedeficiency virus or a parvovirus or a papovavirus, rotavirus or a coronavirus, such as transmissable gastroenteritisvirus or a flavivirus, such as tick-borne encephalitis virus or yellow fever virus, a togavirus, such as rubella virus or eastern-, western-, or venezuelean equine encephalomyelitis virus, a hepatitis causing virus, such as hepatitis A or hepatitis B virus, a pestivirus, such as hog cholera virus or a rhabdovirus, such as rabies virus.
The invention also provides the use of a human PER.C6 cell which cell does not produce structural adenoviral proteins for the production of at least one viral protein for use in a vaccine. Of course for such a use the cells preferred in the methods according to the invention are also preferred. The invention also provides the products resulting from the methods and uses according to the invention, especially viral proteins obtainable according to those uses and/or methods, especially when brought in a pharmaceutical composition comprising suitable excipients and in some formats (subunits) adjuvants. Dosage and ways of administration can be sorted out through normal clinical testing in as far as they are not yet available through the already registered vaccines.
Thus the invention also provides a viral protein for use in a vaccine obtainable by a method or by a use according to the invention, said viral protein being free of any non-human mammalian protenaceous material and a pharmaceutical formulation comprising such a viral protein.
In a preferred embodiment the invention provides influenza vaccines obtainable by a method according to the invention or by a use according to the invention.

### EXAMPLES

To illustrate the invention, the following examples are provided, nut intended to limit the scope of the invention. The human Erythropoietin (EPO) molecule contains four carbohydrate chains. Three contain N-linkages to asparagines, and one contains an O-linkage to a serine residue. The importance of glycosylation in the biological activity of EPO has been well documented (Delorme et al 1992; Yamaguchi et al 1991). The cDNA encoding human EPO was cloned and expressed in PER.C6 cells and PER.C6/E2A cells, expression was shown, and the glycosylation pattern was analyzed.

### Example 1: Construction of basic expression vectors.

Plasmid pcDNA3.1/Hygro(-) (Invitrogen) was digested with NruI and EcoRV, dephosphorylated at the 5' termini by Shrimp Alkaline Phosphatase (SAP, GIBCO Life Tech.) and the plasmid fragment lacking the immediate early enhancer and promoter from cytomegalovirus (CMV) was purified from gel. Plasmid pAdApt, containing the full length CMV enhancer/promoter (-735 to +95) next to overlapping Adeno-derived sequences to produce recombinant Adenovirus, was digested with AvrII, filled in with Klenow polymerase and digested with HpaI; the fragment containing the CMV enhancer and promoter was purified over agarose gel. This CMV enhancer and promoter fragment was ligated blunt/blunt to the NruI/EcoRV fragment from pcDNA3.1/Hygro(-). The resulting plasmid was designated pcDNA2000/Hyg(-).

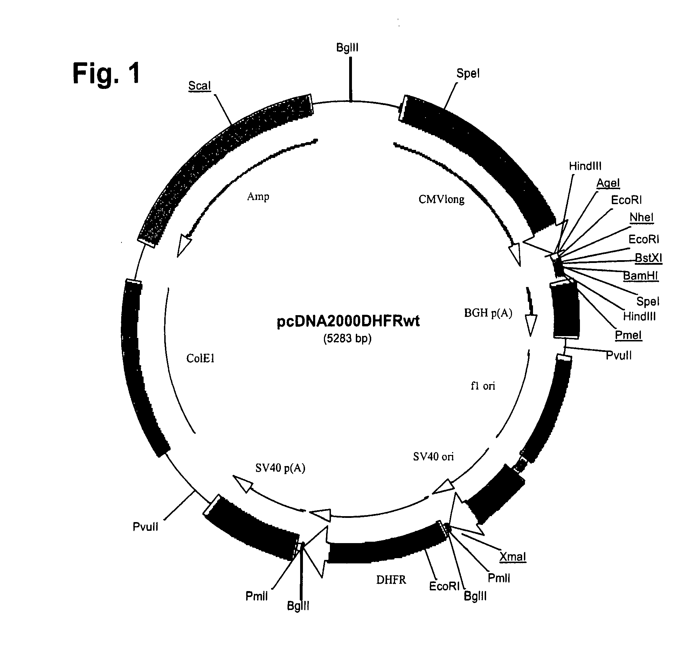

Plasmid pcDNA2000/Hyg(-) was digested with PmlI, and the linearized plasmid lacking the Hygromycine resistance marker gene was purified from gel, and religated. The resulting plasmid was designated pcDNA2000. Plasmid pcDNA2000 was digested with PmlI and dephosphorylated by SAP at both termini. Plasmid pIG-GC9 containing the wild type human DHFR cDNA (Havenga et al 1998) was used to obtain the wild type DHFR-gene by polymerase chain reaction (PCR) with introduced, noncoding, PmlI sites upstream and downstream of the cDNA. PCR primers that were used were DHFR up (SEQ. ID. NO. 1): 5'-GAT CCA CGT GAG ATC TCC ACC ATG GTT GGT TCG CTA AAC TG-3' and DHFR down (SEQ. ID. NO. 2): 5'-GAT CCA CGT GAG ATC TTT AAT CAT TCT TCT CAT ATAC-3'. The PCR-product was digested with PmlI and used for ligation into pcDNA2000 (digested with PmlI, and dephosphorylated by SAP) to obtain pcDNA2000/DHFRwt (Fig 1). Wild type sequences and correctly used cloning sites were confirmed by double stranded sequencing. Moreover, a mutant version of the human DHFR gene (DHFRm) was used to reach a 10,000 fold higher resistance to methotrexate in PER.C6 and PER.C6/E2A by selection of a possible integration of the transgene in a genomic region with high transcriptional activity. This mutant carries an amino acid substitution in position 32 (phenylalanine to serine) and position 159 (leucine to proline) introduced by the PCR procedure. PCR on plasmid pIG-GC12 (Havenga et al 1998) was used to obtain the mutant version of human DHFR. Cloning of this mutant is comparable to wild type DHFR. The plasmid obtained with mutant DHFR was designated pcDNA2000/DHFRm.

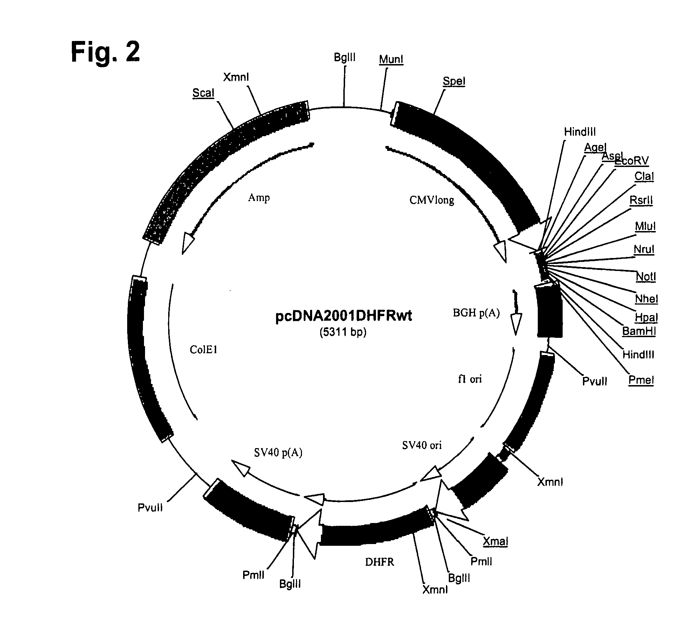

pIPspAdapt 6 (Galapagos) was digested with AgeI and BamHI restriction enzymes. The resulting polylinker fragment has the following sequence (SEQ. ID. NO. 3): 5'-ACC GGT GAA TTC GGC GCG CCG TCG ACG ATA TCG ATC GGA CCG ACG CGT TCG CGA GCG GCC GCA ATT CGC TAG CGT TAA CGG ATC C -3' The used AgeI and BamHI recognition sites are underlined. This fragment contains several unique restriction enzyme recognition sites and was purified over agarose gel and ligated to an AgeI/BamHI digested and agarose gel purified pcDNA2000/DHFRwt plasmid. The resulting vector was named pcDNA2001/DHFRwt (Fig 2)

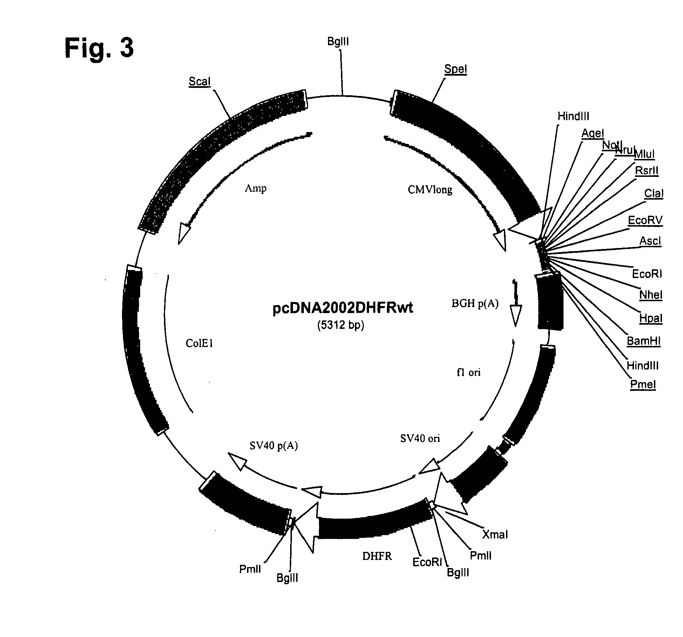

pIPspAdapt7 (Galapagos) is digested with AgeI and BamHI restriction enzymes and has the following sequence (SEQ. ID. NO. 4): 5'- ACC GGT GAA TTG CGG CCG CTC GCG AAC GCG TCG GTC CGT ATC GAT ATC GTC GAC GGC GCG CCG AAT TCG CTA GCG TTA ACG GAT CC-3'. The used AgeI and BamHI recognition sites are underlined. The polylinker fragment contains several unique restriction enzyme recognition sites (different from pIPspAdapt6), which is purified over agarose gel and ligated to an AgeI/BamHI digested and agarose gel purified pcDNA2000/DHFRwt. This results in pcDNA2002/DHFRwt (Fig 3).

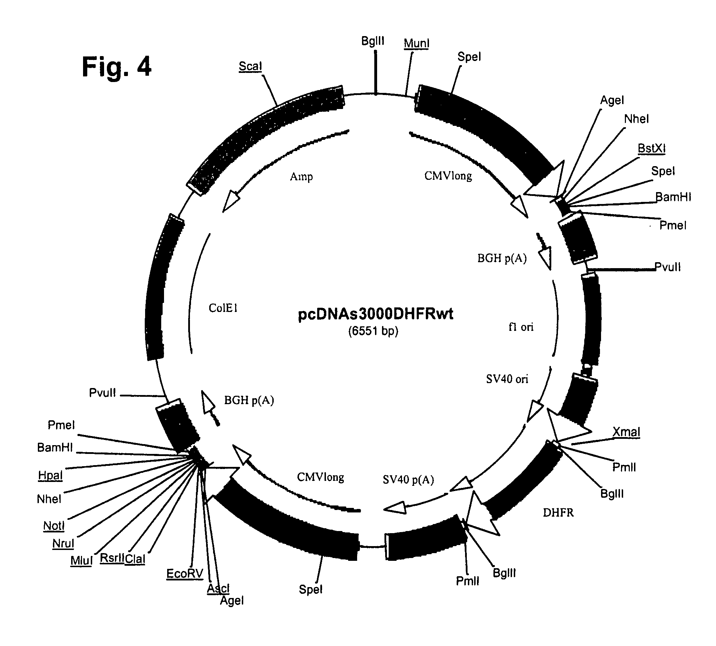

pcDNA2000/DHFRwt was partially digested with restriction enzyme PvuII. There are two PvuII sites present in this plasmid and cloning was performed into the site between the SV40 poly(A) and ColE1, not the PvuII site downstream of the BGH poly(A). A single site digested mixture of plasmid was dephosphorylated with SAP and blunted with Klenow enzyme and purified over agarose gel. pcDNA2001/DHFRwt was digested with MunI and PvuII restriction enzymes and filled in with Klenow and free nucleotides to have both ends blunted. The resulting CMV promoter-linker-BGH poly(A)-containing fragment was isolated over gel and separated from the vector. This fragment was ligated into the partially digested and dephosphorylated vector and checked for orientation and insertion site. The resulting plasmid was named pcDNAs3000/DHFRwt (Fig 4).

### Example 2 : Construction of EPO expression vectors.

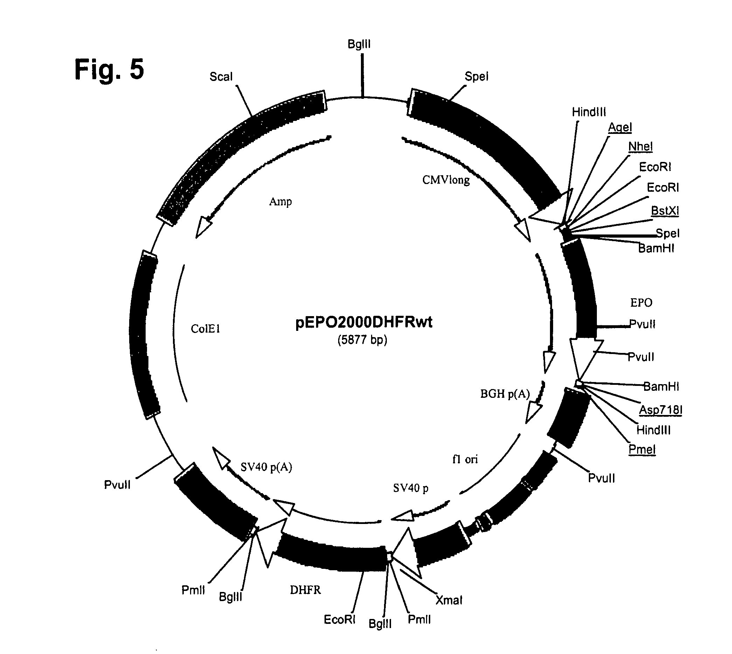

The full length human EPO cDNA was cloned, employing oligonucleotide primers EPO-START (SEQ. ID. NO. 5):5' AAA AAG GAT CCG CCA CCA TGG GGG TGC ACG AAT GTC CTG CCT G-3' and EPO-STOP (SEQ. ID. NO. 6):5'AAA AAG GAT CCT CAT CTG TCC CCT GTC CTG CAG GCC TC-3' (Cambridge Bioscience Ltd) in a PCR on a human adult liver cDNA library. The amplified fragment was cloned into pUC18 linearized with BamHI. Sequence was checked by double stranded sequencing. This plasmid, containing the EPO cDNA in pUC18 was digested with BamHI and the EPO insert was purified from agarose gel. Plasmids pcDNA2000/DHFRwt and pcDNA2000/DHFRm were linearized with BamHI and dephosphorylated at the 5' overhang by SAP, and the plasmids were purified from agarose gel. The EPO cDNA fragment was ligated into the BamHI sites of pcDNA2000/DHFRwt and pcDNA2000/DHFRm; the resulting plasmids were designated pEP02000/DHFRwt (Fig 5) and pEP02000/DHFRm.

### Example 3: Construction of UBS-54 expression vectors

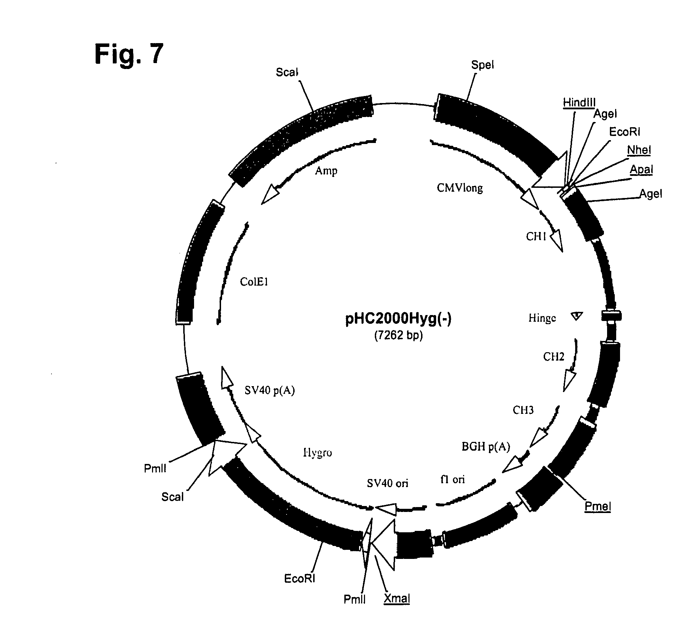

The constant domains (CH1, -2 and -3) of the heavy chain of the human immunoglobulin G1 (IgG1) gene including intron sequences and connecting ('Hinge') domain were generated by PCR using an upstream and a downstream primer. The sequence of the upstream primer (CAMH-UP; SEQ. ID. NO. 17) is 5'-GAT CGA TAT CGC TAG CAC *CAA GGG CCC ATC GGT C*-3'*,* in which the annealing nucleotides are depicted in italic and two sequential restriction enzyme recognition sites (EcoRV and NheI) are underlined. The sequence of the downstream primer (CAMH-DOWN; SEQ. ID. NO. 18) is: 5'-GAT CGT TTA AAC *TCA TTT ACC CGG AGA CAG*-3'*,* in which the annealing nucleotides are depicted in *italic* and the introduced PmeI restriction enzyme recognition site is underlined. The order in which the domains of the human IgG1 heavy chain were arranged are as follows: CHl-intron-Hinge-intron-CH2-intron-CH3. The PCR was performed on a plasmid (pCMgamma NEO Skappa Vgamma Cgamma hu) containing the heavy chain of a humanized antibody directed against D-dimer from human fibrinogen (Vandamme et al. 1990). This antibody was named 15C5 and the humanization was performed with the introduction of the human constant domains including intron sequences (Bulens et al. 1991).
The PCR resulted in a product of 1621 nucleotides. The NheI and PmeI sites were introduced for easy cloning into the pcDNA2000/Hyg(-) polylinker. The NheI site encoded two amino acids (Ala and Ser), that are part of the constant region CH1, but that did not hybridize to the DNA present in the template (Crowe et al. 1992).
The PCR product was digested with NheI and PmeI restriction enzymes, purified over agarose gel and ligated into a NheI and PmeI digested and agarose gel purified pcDNA2000/Hygro(-). This resulted in plasmid pHC2000/Hyg(-) (Fig 7), which can be used for linking the human heavy chain constant domains, including introns to any possible variable region of any identified immunoglobulin heavy chain for humanization.

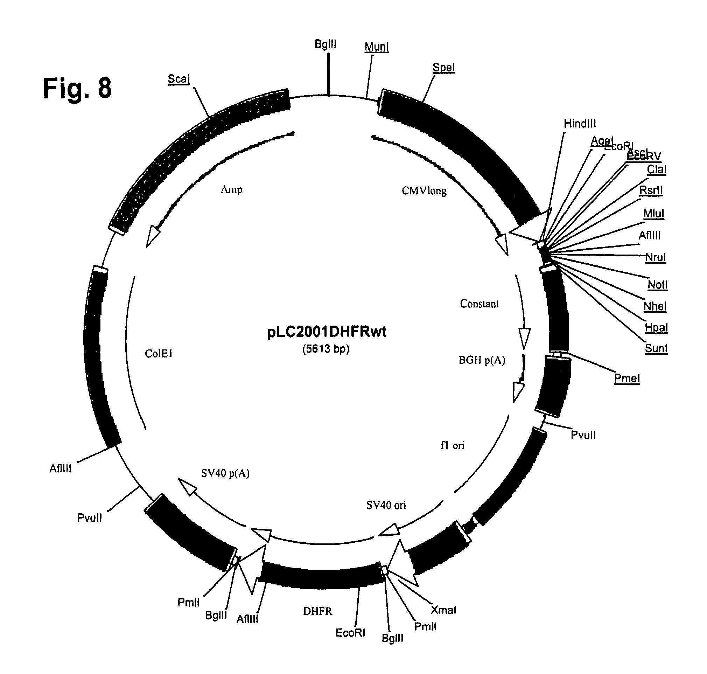

The constant domain of the light chain of the human immunoglobulin (IgG1) gene was generated by PCR using an upstream and a downstream primer: The sequence of the upstream primer (CAML-UP; SEQ. ID. NO. 19) is 5'-GAT CCG TAC G*GT GGC TGC ACC ATC TGT C*-3'*,* in which the annealing nucleotides are depicted in italic and an introduced SunI restriction enzyme recognition site is underlined. The sequence of the downstream primer (CAML-DOWN; SEQ. ID. NO. 20) is 5'-GAT CGT TTA AAC *CTA ACA CTC TCC CCT GTT G*-3', in which the annealing nucleotides are in italic and an introduced Pmel restriction enzyme recognition site is underlined.
The PCR was performed on a plasmid (pCMkappa DHFR13 15C5 kappa humanized) carrying the murine signal sequence and murine variable region of the light chain of 15C5 linked to the constant domain of the human IgG1 light chain (Vandamme et al. 1990; Bulens et al. 1991).
The PCR resulted in a product of 340 nucleotides. The SunI and PmeI sites were introduced for cloning into the pcDNA2001/DHFRwt polylinker. The SunI site encoded two amino acids (Arg and Thr) of which the threonine residue is part of the constant region of human immunoglobulin light chains, while the arginine residue is part of the variable region of CAMPATH-1H (Crowe et al. 1992). This enabled subsequent 3' cloning into the SunI site, which was unique in the plasmid.
The PCR product was digested with SunI and PmeI restriction enzymes purified over agarose gel, ligated into a BamHI, PmeI digested, and agarose gel purified pcDNA2001/DHFRwt, which was blunted by Klenow enzyme and free nucleotides. Ligation in the correct orientation resulted in loss of the BamHI site at the 5' end and preservation of the SunI and PmeI sites. The resulting plasmid was named pLC2001/DHFRwt (Fig 8) which plasmid can be used for linking the human light chain constant domain to any possible variable region of any identified immunoglobulin light chain for humanization.

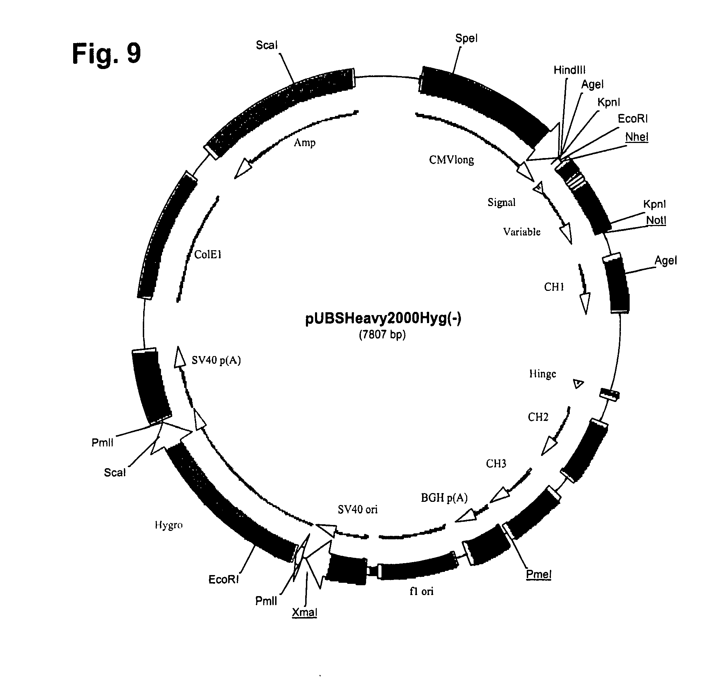

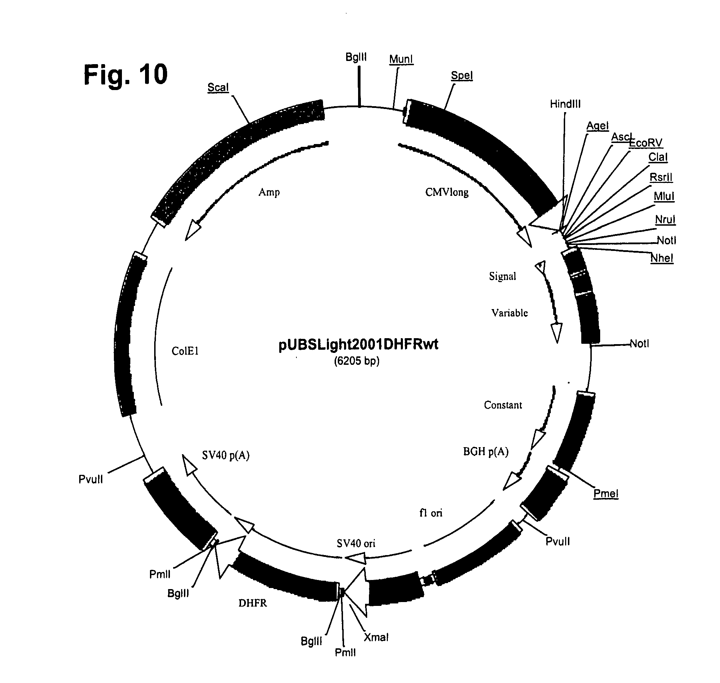

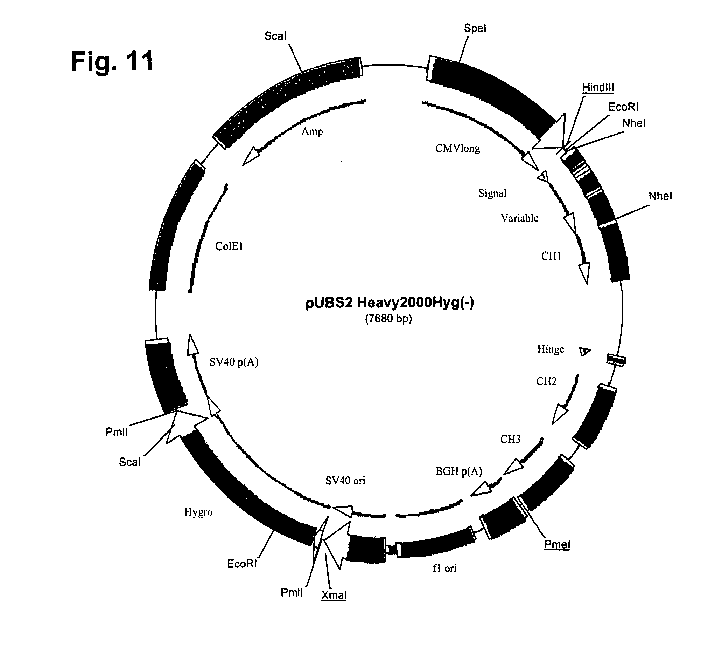

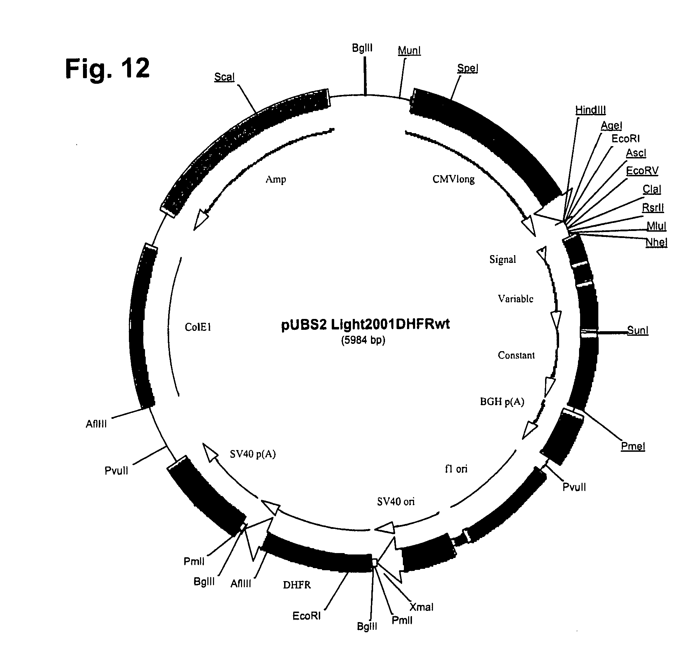

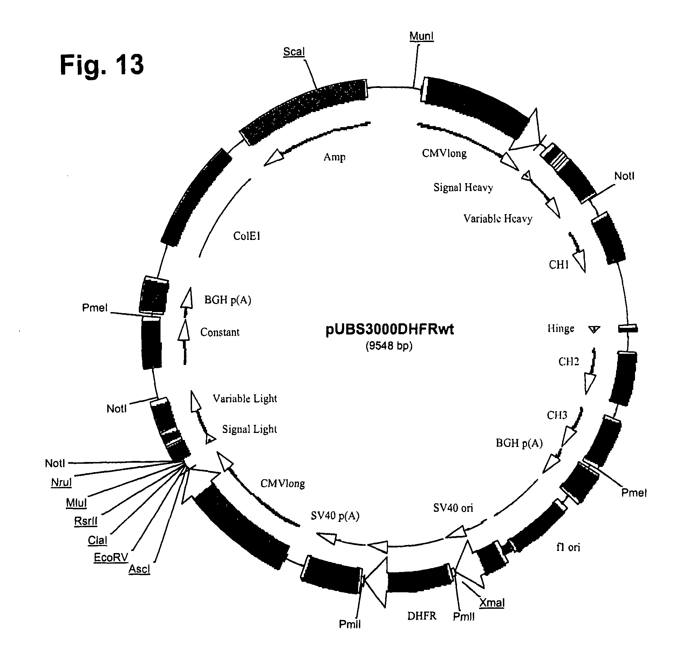

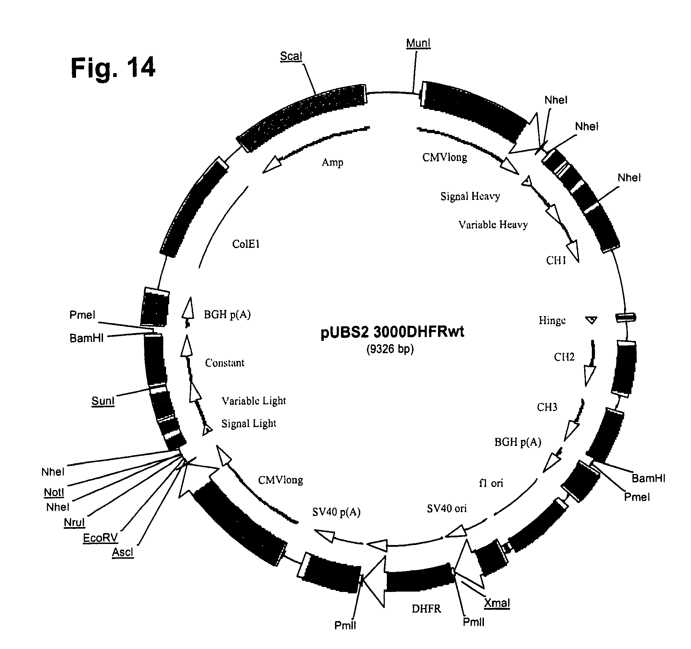

pNUT-C gamma (Huls et al., 1999) contains the constant domains, introns and hinge region of the human IgG1 heavy chain (Huls et al. 1999) and received upstream of the first constant domain the variable domain of the gamma chain of fully humanized monoclonal antibody UBS-54 preceded by the following leader peptide sequence (SEQ. ID. NO. 21): MACPGFLWALVISTCLEFSM (sequence (SEQ. ID. NO. 22): 5'- ATG GCA TGC CCT GGC TTC CTG TGG GCA CTT GTG ATC TCC ACC TGT CTT GAA TTT TCC ATG-3'). This resulted in an insert of approximately 2 kb in length. The entire gamma chain was amplified by PCR using an upstream primer (UBS-UP) and the downstream primer CAMH-DOWN. The sequence of UBS-UP (SEQ. ID. NO. 23) is as follows: 5'-GAT CAC GCG TGC TAG *CCA CCA TGG* CAT GCC CTG GCT TC-3' in which the introduced MIuI and NheI sites are underlined and the perfect Kozak sequence is italicized.
The resulting PCR product was digested with NheI and Pmel restriction enzymes, purified over agarose gel and ligated to the pcDNA2000/Hygro(-) plasmid that is also digested with NheI and PmeI, dephosphorylated with tSAP and purified over gel. The resulting plasmid was named pUBS-Heavy2000/Hyg(-) (Fig 9). pNUT-C kappa contains the constant domain of the light chain of human IgG1 kappa (Huls et al. 1999) and received the variable domain of fully humanized monoclonal antibody UBS-54 kappa chain preceded by the following leader peptide: MACPGFLWALVISTCLEFSM (sequence: 5'- ATG GCA TGC CCT GGC TTC CTG TGG GCA CTT GTG ATC TCC ACC TGT CTT GAA TTT TCC ATG -3', for details on the plasmid see UBiSys). This resulted in an insert of approximately 1.2 kb in length.
The entire insert was amplified by PCR using the upstream primer UBS-UP and the downstream primer CAML-DOWN, hereby modifying the translation start site. The resulting PCR product was digested with NheI and PmeI restriction enzymes, purified over agarose gel and ligated to pcDNA2001/DHFRwt that was also digested with NheI and PmeI, dephosphorylated by tSAP and purified over gel, resulting in pUBS-Light2001/DHFRwt (Fig 10). To remove the extra intron which is located between the variable domain and the first constant domain that is present in pNUT-Cgamma and to link the signal peptide and the variable domain to the wild type constant domains of human IgG1 heavy chain, lacking a number of polymorphisms present in the carboxy-terminal constant domain in pNUT-Cgamma, a PCR product is generated with primer UBS-UP and primer UBSHV-DOWN that has the following sequence (SEQ. ID. NO. 24): 5'- GAT CGC TAG *CTG TCG AGA CGG TGA CCA* G -3', in which the introduced NheI site is underlined and the annealing nucleotides are italicized. The resulting PCR product is digested with Nhel restriction enzyme, purified over gel and ligated to a NheI digested and SAP-dephosphorylated pHC2000/Hyg(-) plasmid that was purified over gel. The plasmid with the insert in the correct orientation and reading frame is named pUBS2-Heavy2000/Hyg(-) (Fig 11).
For removal of an extra intron which is located between the variable domain and the constant domain that is present in pNUT-Ckappa and to link the signal peptide and the variable domain to the wild type constant domain of human IgG1 light chain, a PCR product was generated with primer UBS-UP and primer UBSLV-DOWN that has the following sequence (SEQ. ID. NO. 25): 5'- GAT CCG TAC GCT TGA TCT CCA CCT TGG TC -3', in which the introduced SunI site is underlined. Then the resulting PCR product was digested with MluI and SunI restriction enzymes, purified over gel and ligated to a MluI and SunI digested pLC2001/DHFRwt plasmid that was purified over gel. The resulting plasmid was named pUBS2-Light2001/DHFRwt (Fig 12). The PCR product of the full-length heavy chain of UBS-54 is digested with NheI and PmeI restriction enzymes and blunted with Klenow enzyme. This fragment is ligated to the plasmid pcDNAs3000/DHFRwt that is digested with BstXI restriction enzyme, blunted, dephosphorylated by SAP and purified over gel. The plasmid with the heavy chain insert is named pUBS-Heavy3000/DHFRwt. Subsequently, the PCR of the light chain is digested with MluI and PmeI restriction enzymes, blunted, purified over gel and ligated to pUBS-Heavy3000/DHFRwt that is digested with HpaI, dephosphorylated by tSAP and purified over gel. The resulting vector is named pUBS-3000/DHFRwt (Fig 13). The gene that encodes the heavy chain of UBS-54 without an intron between the variable domain and the first constant region and with a wild type carboxy terminal constant region (2031 nucleotides) is purified over gel after digestion of pUBS2-2000/Hyg(-) with EcoRI and PmeI and treatment with Klenow enzyme and free nucleotides to blunt the EcoRI site. Subsequently, the insert is ligated to a pcDNAs3000/DHFRwt plasmid that is digested with BstXI, blunted, dephosphorylated with SAP and purified over gel. The resulting plasmid is named pUBS2-Heavy3000/DHFRwt. pUBS2-Light2001/DHFRwt is then digested with EcoRV and PmeI, and the 755 nucleotide insert containing the signal peptide linked to the variable domain of the kappa chain of UBS-54 and the constant domain of human IgG1 kappa chain without an intron sequence is purified over gel and ligated to pUBS2-Heavy3000/DHFRwt that is digested with Hpal, dephosphorylated with tSAP and purified over gel. The resulting plasmid is named pUBS2-3000/DHFRwt (Fig 14).

Plasmid pRc/CMV (Invitrogen) was digested with BstBI restriction enzymes, blunted with Klenow enzyme and subsequently digested with Xmal enzyme. The Neomycin resistance gene containing fragment was purified over agarose gel and ligated to pUBS-Light2001/DHFRwt plasmid that was digested with Xmal and PmlI restriction enzymes, followed by dephosphorylation with SAP and purified over gel to remove the DHFR cDNA. The resulting plasmid was named pUBS-Light2001/Neo. The fragment was also ligated to a XmaI/PmlI digested and gelpurified pcDNA2001/DHFRwt plasmid resulting in pcDNA2001/Neo. The PCR product of the UBS-54 variable domain and the digested and purified constant domain PCR product were used in a three-point ligation with a MluI/PmeI digested pcDNA2001/Neo. The resulting plasmid was named pUBS2-light2001/Neo.

### Example 4: Construction of CAMPATH-1H expression vectors

Cambridge Bioscience Ltd. (UK) generates a 396 nucleotide fragment containing a perfect Kozak sequence followed by the signal sequence and the variable region of the published CAMPATH-1H light chain (Crowe et al. 1992). This fragment contains, on the 5' end, an introduced and unique HindIII site and, on the 3' end, an introduced and unique SunI site and is cloned into an appropriate shuttle vector. This plasmid is digested with HindIII and SunI and the resulting CAMPATH-1H light chain fragment is purified over gel and ligated into a HindIII/SunI digested and agarose gel purified pLC2001/DHFRwt. The resulting plasmid is named pCAMPATH-Light2001/DHFRwt. Cambridge Bioscience Ltd. (UK) generated a 438 nucleotide fragment containing a perfect Kozak sequence followed by the signal sequence and the published variable region of the CAMPATH-1H heavy chain (Crowe et al. 1992), cloned into an appropriate cloning vector. This product contains a unique HindIII restriction enzyme recognition site on the 5' end and a unique NheI restriction enzyme recognition site on the 3' end. This plasmid was digested with HindIII and NheI and the resulting CAMPATH-1H heavy chain fragment was purified over gel and ligated into a purified and HindIII/NheI digested pHC2000/Hyg(-). The resulting plasmid was named pCAMPATH-Heavy2000/Hyg(-).

### Example 5

### Construction of 15C5 expression vectors

The heavy chain uf the humanized version of the monoclonal antibody 15C5 directed against human fibrin fragment D-dimer (Bulens et al. 1991; Vandamme et al. 1990) consisting of human constant domains including intron sequences, hinge region and variable regions preceded by the signal peptide from the 15C5 kappa light chain is amplified by PCR on plasmid "pCMgamma NEO Skappa Vgamma Cgamma hu" as a template using CAMH-DOWN as a downstream primer and 15C5-UP as upstream primer. 15C5-UP has the following sequence (SEQ. ID. NO. 26): 5'- GA TCA CGC GTG CTA *GCC ACC ATG G*GT ACT CCT GCT CAG TTT CTT GGA ATC -3', in which the introduced MluI and NheI restriction recognition sites are underlined and the perfect Kozak sequence is italicized. To properly introduce an adequate Kozak context, the adenine at position +4 (the adenine in the ATG start codon is +1) is replaced by a guanine resulting in a mutation from an arginine into a glycine amino acid. To prevent primer dimerization, position +6 of the guanine is replaced by a thymine and the position +9 of the cytosine is replaced by thymine. This latter mutation leaves the threonine residue intact. The resulting PCR was digested with NheI and PmeI restriction enzymes, purified over gel and ligated to a NheI and PmeI digested pcDNA2000/Hygro(-), that is dephosphorylated by SAP and purified over agarose gel. The resulting plasmid is named p15C5-Heavy2000/Hyg(-). The light chain of the humanized version of the monoclonal antibody 15C5 directed against human fibrin fragment D-dimer (Bulens et al. 1991; Vandamme et al. 1990) consisting of the human constant domain and variable regions preceded by a 20 amino acid signal peptide is amplified by PCR on plasmid pCMkappa DHFR13 15C5kappa hu as a template, using CAML-DOWN as a downstream primer and 15C5-UP as the upstream primer. The resulting PCR is digested with NheI and PmeI restriction enzymes, purified over gel and ligated to a NheI and PmeI digested pcDNA2001/DHFRwt that is dephosphorylated by SAP and purified over agarose gel. The resulting plasmid is named p15C5-Light2001/DHFRwt.

### Example 6: Establishment of methotrexate hygromycin and G418 selection levels.

PER.C6 and PER.C6/E2A were seeded in different densities. The starting concentration of methotrexate (MTX) in these sensitivity studies ranged between O nM and 2500nM. The concentration which was just lethal for both cell lines was determined; when cells were seeded in densities of 100.000 cells per well in a 6-wells dish, wells were still 100% confluent at 10nM, approximately 90-100% confluent at 25nM, while most cells were killed at concentration at 50nM and above after 6 days to 15 days of incubation. These results are summarized in table 1. PER.C6 cells were tested for their resistance to a combination of Hygromycin and G418 to select outgrowing stable colonies that expressed both heavy and light chains for the respective recombinant monoclonal antibodies encoded by plasmids carrying either a hygromycin or a neomycin resistance gene. When cells were grown on normal medium containing 100ug/ml hygromycin and 250 ug/ml G418 non-transfected cells were killed and stable colonies could appear (see example 7)

CHO-dhfr cells ATCC:CRL9096 are seeded in different densities in their respective culture medium. The starting concentration of methotrexate in these sensitivity studies ranges from approximately 0.5 nM to 500 nM. The concentration, which is just lethal for the cell line, is determined, and subsequently used directly after growth selection on hygromycin in the case of IgG heavy chain selection (hyg) and light chain selection (dhfr).

### Example 7: Transfection of EPO expression vectors to obtain stable cell lines.

Cells of cell lines PER.C6 and PER.C6/E2A were seeded in 40 tissue culture dishes (10 cm diameter) with approximately 2-3 million cells/dish and were kept overnight under their respective conditions (10% CO₂ concentration and temperature, which is 39°C for PER.C6/E2A and 37°C for PER.C6). On the next day, transfections were all performed at 37°C using Lipofectamine (Gibco). After replacement with fresh (DMEM) medium after 4 hours, PER.C6/E2A cells were transferred to 39°C again, while PER.C6 cells were kept at 37°C. Twenty dishes of each cell line were transfected with 5 ug ScaI digested pEP02000/DHFRwt and twenty dishes were transfected with 5 ug ScaI digested pEPO2000/DHFRm all according to standard protocols. Another 13 dishes served as negative controls for methotrexate killing and transfection efficiency which was approximately 50%. On the next day, MTX was added to the dishes in concentrations ranging between 100 and 1000 nM for DHFRwt and 50.000 and 500.000nM for DHFRm dissolved in medium containing dialyzed FBS. Cells were incubated over a period of 4-5 weeks. Tissue medium (including MTX) was refreshed every two-three days. Cells were monitored daily for death, comparing between positive and negative controls. Outgrowing colonies were picked and subcultured. No positive clones could be subcultured from the transfectants that received the mutant DHFR gene most likely due to toxic effects of the high concentrations of MTX that were applied. From the PER.C6 and PER.C6/E2A cells that were transfected with the wild type DHFR gene, only cell lines could be established in the first passages when cells were grown on 100nM MTX, although colonies appeared on dishes with 250 and 500 nM MTX. These clones were not viable during subculturing, and were discarded.

### Example 8: Subculturing of transfected cells.

From each cell line, approximately 50 selected colonies that were resistant to the threshold MTX concentration were grown subsequently in 96-wells, 24-wells, 6-wells plates and T25 flask in their respective medium plus MTX. When cells reached growth in T25 tissue culture flasks at least one vial of each clone was frozen and stored, and was subsequently tested for human recombinant EPO production. For this, the commercial ELISA kit from R&D Systems was used (Quantikine IVD human EPO, Quantitative Colorimetric Sandwich ELISA, cat.# DEPOO). Since the different clones appeared to have different growth characteristics and growth curves, a standard for EPO production was set as fellows: At day 0 cells were seeded in T25 tissue culture flasks in concentrations ranging between 0.5 to 1.5 million per flask. At day 4. Supernatant was taken and used in the EPO ELISA. From this the production level was set as ELISA units per million seeded cells per day. (U/1E6/day) A number of these clones are given in table 2.
The following selection of good producer clones was based on high expression, culturing behaviour and viability. To allow checks for long term viability, suspension growth in roller bottles and bioreactor during extended time periods, more vials of the best producer clones where frozen, and the following best producers of each cell line were selected for further investigations P8, P9, E17 and E55 in which "P" stands for PER.C6 and "E" stands for PER.C6/E2A. These clones are subcultured and subjected to increasing doses of methotrexate in a time span of two months. The concentration starts at the threshold concentration and increases to approximately 0.2 mM. During these two months, EPO ELISA experiments are performed on a regular basis to detect an increase in EPO production. At the highest methotrexate concentration, the best stable producer is selected and compared to the amounts from the best CHO clone and used for cell banking (RL). From every other clone 5 vials are frozen. The number of amplified EPO cDNA copies is detected by Southern blotting.

### Example 9: EPO production in bioreactors

The best performing EPO producing transfected stable cell line of PER.C6, P9, was brought into suspension and scaled up to 1 to 2 liter fermentors. To get P9 into suspension, attached cells were washed with PBS and subsequently incubated with JRH ExCell 525 medium for PER.C6 (JRH), after which the cells losen from the flask and form the suspension culture. Cells were kept at two concentrations of MTX: 0 nM and 100 nM. General production levels of EPO that were reached at these concentrations (in rollerbottles) were respectively 1500 and 5700 units per million seeded cells per day. Although the lower yields in the absence of MTX can be explained by removal of the integrated DNA, it seems as if there is a shut-down effect of the integrated DNA since cells that are kept at lower concentrations of MTX for longer periods of time are able to reach their former yields when they are transferred to 100 nM MTX concentrations again (see example 11).
Suspension P9 cells were grown normally with 100 nM MTX and used for inoculation of bioreactors. Two bioreactor settings were tested: Perfusion and repeated batch cultures.

### A. Perfusion in a 2 liter bioreactor.

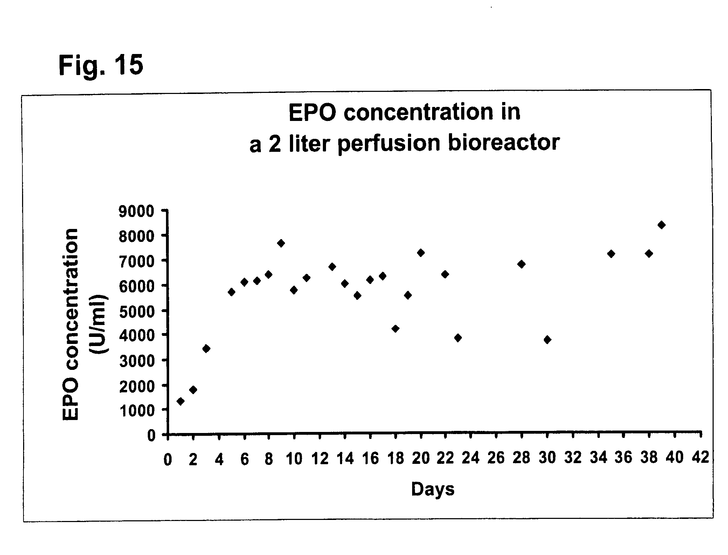

Cells were seeded at a concentration of 0.5 x 10⁶ cells per ml and perfusion was started at day 3 after cells reached a density of approximately 2.3 x 10⁶ cells per ml. The perfusion rate was I volume per 24 hours with a bleed of approximately 250 ml per 24 hours. In this setting, P9 cells stayed at a constant density of approximately 5 x 10⁶ cells per ml and a viability of almost 95% for over a month. The EPO concentration was determined on a regular basis and shown in Figure 15. In the 2 liter perfused bioreactor the P9 cells were able to maintain a production level of approximately 6000 ELISA units per ml. With a perfusion rate of 1 working volume per day (1.5 to 1.6 liter), this means that in this 2 liter setting the P9 cells produced approximately 1 x 10⁷ units per day per 2 liter bioreactor in the absence of MTX.

### B. Repeated batch in a 2 liter bioreactor.

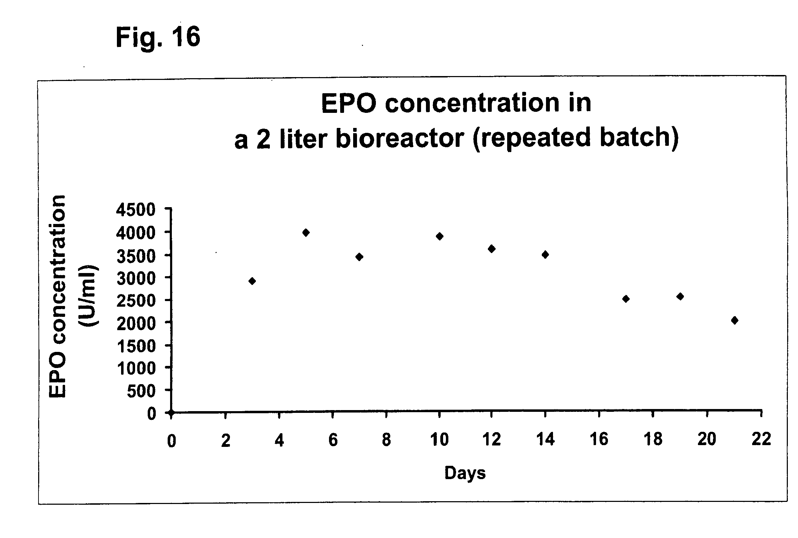

P9 suspension cells that were grown on rollerbottles were used to inoculate a 2 liter bioreactor in the absence of MTX and were left to grow till a density of approximately 1.5 million cells per ml after which a third of the population was removed (± 1 liter per 2 to 3 days) and the remaining culture was diluted with fresh medium to reach again a density of 0.5 million cells per ml. This procedure was repeated for 3 weeks and the working volume was kept at 1.6 liter. EPO concentrations in the removed medium was determined and shown in Figure 16. The average concentration was approximately 3000 ELISA units per ml. With an average period of 2 days after which the population was diluted, this means that in this 2 liter setting the P9 cells produced approximately 1.5 x 10⁶ units per day in the absence of MTX.

### C. Repeated batch in a 1 liter bioreactor with different concentrations dissolved oxygen, temperatures and pH settings.

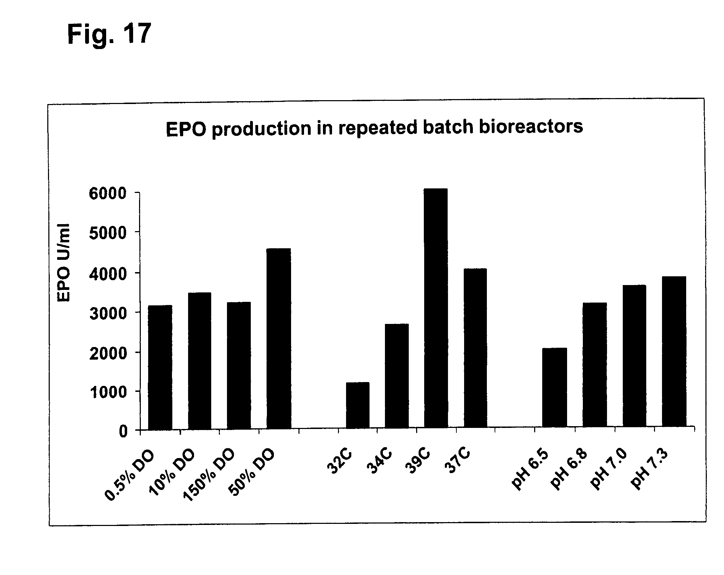

Fresh P9 suspension cells were grown in the presence of 100 nM MTX in rollerbottles and used for inoculation of 4 x 1 liter bioreactors to a density of 0.3 million cells per ml in JRH ExCell 525 medium. EPO yields were determined after 3, 5 and 7 days. The first settings that were tested were: 0.5%, 10%, 150% and as a positive control 50% Dissolved Oxygen (%DO). 50% DO is the condition in which PER.C6 and P9 cells are normally kept. In another run, P9 cells were inoculated and tested for EPO production at different temperatures (32°C, 34°C, 37°C and 39°C) in which 37°C is the normal setting for PER.C6 and P9, and in the third run fresh P9 cells were inoculated and tested for EPO production at different pH settings (pH 6.5, pH 6.8, pH 7.0 and pH 7.3). PER.C6 cells are normally kept at pH 7.3. An overview of the EPO yields (three days after seeding) is shown in Figure 17. Apparently, EPO concentrations increase when the temperature is rising from 32 to 39°C as was also seen with PER.C6/E2A cells grown at 39°C (Table 4), and 50% DO is optimal for P9 in the range that was tested here. At pH 6.5, cells can not survive since the viability in this bioreactor dropped beneath 80% after 7 days. EPO samples produced in these settings are checked for glycosylation and charge in 2D electrophoresis (see also example 17).

### Example 10: Amplification of the DHFR gene

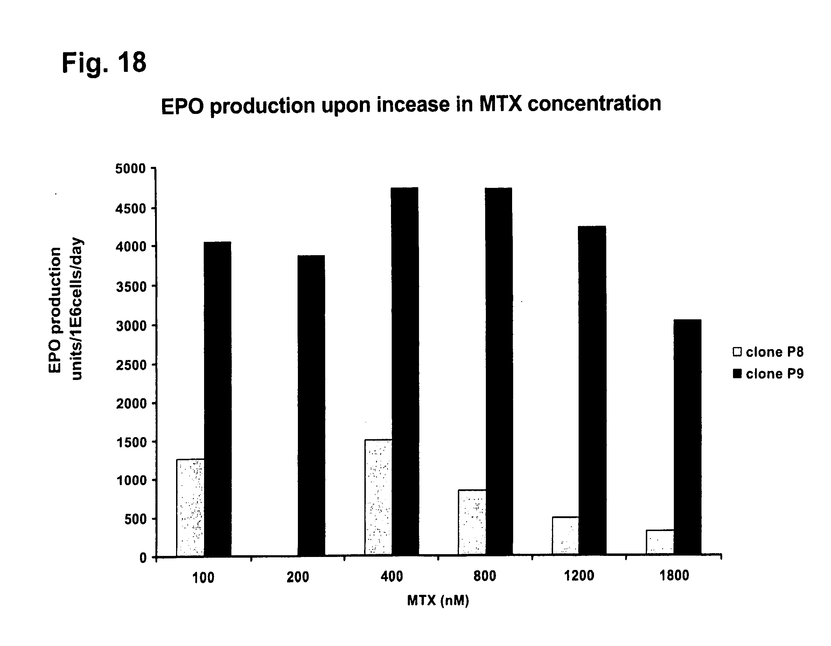

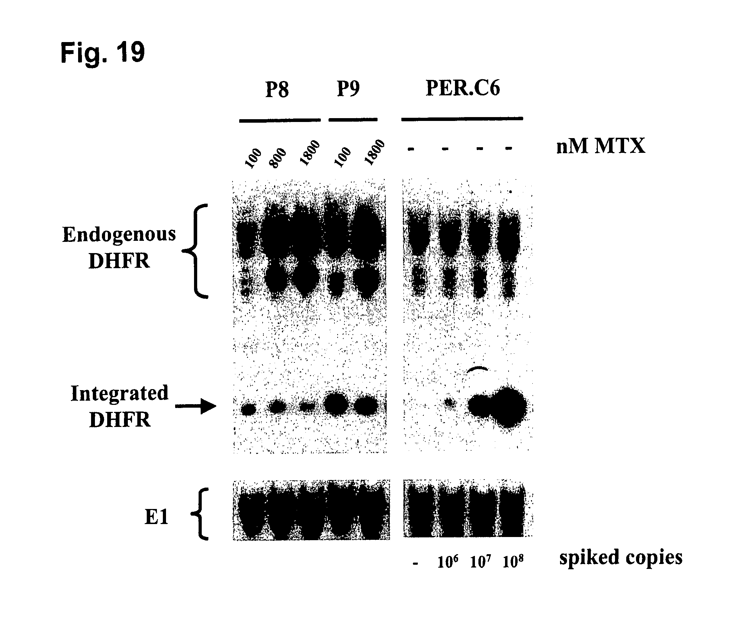

A number of cell lines described in example 8 were used in an amplification experiment to determine the possibility of increasing the number of DHFR genes by increasing the concentration of MTX in a time span of more than two months. The concentration started at the threshold concentration (100 nM) and increased to 1800 nM with in-between steps of 200 nM, 400 nM, 800 nM and 1200 nM. During this period, EPO ELISA experiments were performed on a regular basis to detect the units per million seeded cells per day (Fig. 18). At the highest MTX concentration (1800 nM), some vials were frozen. Cell pellets were obtained and DNA was extracted and subsequently digested with BglII, since this enzyme cuts around the wild type DHFR gene in pEPO2000/DHFRwt (Fig. 5), so a distinct DHFR band of that size would be distinguishable from the endogenous DHFR bands in a Southern blot. This DNA was run and blotted and the blot was hybridized with a radioactive DHFR probe and subsequently with an adenovirus E1 probe as a background control (Fig. 19). The intensities of the hybridizing bands were measured in a phosphorimager and corrected for background levels. These results are shown in table 3. Apparently it is possible to obtain amplification of the DHFR gene in PER.C6 cells albeit in this case only with the endogenous DHFR and not with the integrated vector.

### Example 11: Stability of EPO expression in stable cell lines

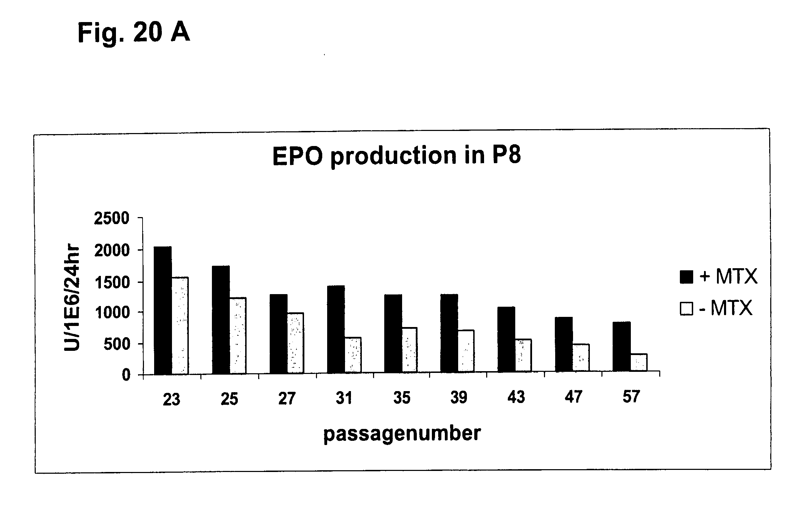

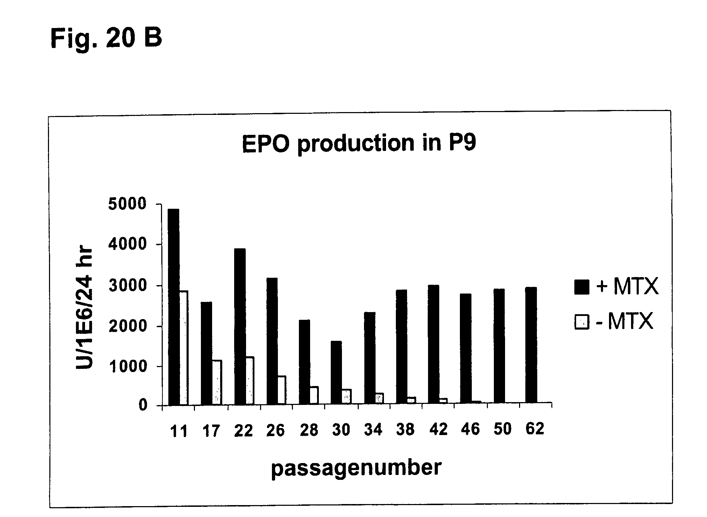

A number of cell lines mentioned in example 8 were subject to long term culturing in the presence and absence of MTX. EPO concentrations were measured regularly in which 1.0 to 1.5 x 10⁶ cells per T25 flask were seeded and left for 4 days to calculate the production levels of EPO per million seeded cells per day. The results are shown in Figure 20. From this it is concluded that there is a relatively stable expression of EPO in P9 cells when cells are cultured in the presence of MTX and that there is a decrease in EPO production in the absence of MTX. However, when P9 cells were placed on 100 nM MTX again after being cultured for a longer period of time without MTX, the expressed EPO reached it original level (±3000 ELISA units per million seeded cells per day), suggesting that the integrated plasmids are shut off but are stably integrated and can be switched back on again. It seems as if there are differences between the cell lines P8 and P9 because the production level of P8 in the presence of MTX is decreasing in time over a high number of passages (Fig. 20A), while P9 production is stable for at least 62 passages (Fig. 20B).

### Example 12: Transient expression of recombinant EPO on attached and suspension cells after plasmid DNA transfections

pEP02000/DHFRwt pEP02000/DHFRm and pAdApt.EPO plasmids from example 2 are purified from E. coli over columns, and are transfected using Lipofectamine, electroporation, PEI or other methods. PER.C6 or PER.C6/E2A cells are counted and seeded in DMEM plus serum or JRH ExCell 525 medium or the appropriate medium for transfection in suspension. Transfection is performed at 37°C up to 16 hours depending on the transfection method used, according to procedures known by a person skilled in the art. Subsequently the cells are placed at different temperatures and the medium is replaced by fresh medium with or without serum. In the case when it is necessary to obtain medium that completely lacks serum components, the fresh medium lacking serum is removed again after 3 hours and replaced again by medium lacking serum components. For determination of recombinant EPO production, samples are taken at different time points. Yields of recombinant protein is determined using an ELISA kit (R&D Systems) in which 1 Unit equals approximately 10 ng of recombinant CHO- produced EPO protein (100,000 Units/mg). The cells used in these experiments grow at different rates, due to their origin, characteristics and temperature. Therefore, the amount of recombinant EPO produced is generally calculated in ELISA units/10⁶ seeded cells/day, taking into account that the antisera used in the ELISA kit do not discriminate between non- and highly glycosylated recombinant EPO. Generally, samples for these calculations are taken at day 4 after replacing the medium upon transfection.

PER.C6/E2A cells, transfected at 37°C using Lipofectamine and subsequently grown at 39°C in the presence of serum produced typically 3100 units/10⁶ cells/day. In the absence of serum components without any refreshment of medium lacking serum these Lipofectamine-transfected cells produced typically 2600 units/10⁶ cells/day. PER.C6 cells, transfected at 37°C using Lipofectamine and subsequently grown at 37°C in the presence of serum produced typically 750 units/10⁶ cells/day, and in the absence of serum 590 units/10⁶ cells/day. For comparison, the same expression plasmids pEP02000/DHFRwt and pEPO2000/DHFRm, were also applied to transfect Chinese Hamster Ovary cells (CHO, ECACC nr.85050302) using Lipofectamine, PEI, Calcium Phosphate procedures and other methods. When CHO cells were transfected using lipofectamine and subsequently cultured in Hams F12 medium in the presence of serum, a yield of 190 units/10⁶ cells/day was obtained. In the absence of serum 90 units/10⁶ cells/day was produced, although higher yields can be obtained when transfections are being performed in DMEM. Different plates containing attached PER.C6/E2A cells were also transfected at 37°C with pEPO2000/DHFRwt plasmid and subsequently placed at 32°C, 34°C, 37°C or 39°C to determine the influence of temperature on recombinant EPO production. A temperature dependent production level was observed ranging from 250 to 610 units/10⁶ seeded cells/day, calculated from a day 4 sample, suggesting that the difference between production levels observed in PER.C6 and PER.C6/E2A is partly due to incubation temperatures (see also Fig 17). Since PER.C6/E2A grows well at 37°C, further studies were performed at 37°C.

Different plates containing attached PER.C6 and PER.C6/E2A cells were transfected with pEP02000/DHFRwt, pEPO2000/DHFRm and pAdApt.EPO using Lipofectamine. Four hours after transfection the DMEM was replaced with either DMEM plus serum or JRH medium lacking serum and EPO was allowed to accumulate in the supernatant for several days to determine the concentrations that are produced in the different mediums. PER.C6 cells were incubated at 37°C, while PER.C6/E2A cells were kept at 39°C. Data from the different plasmids were averaged since they contain a similar expression cassette. Calculated from a day 6 sample the following data was obtained: PER.C6 grown in DMEM produced 400 units/10⁶ seeded cells/day, and when they were kept in JRH medium they produced 300 units/10⁶ seeded cells/day. PER.C6/E2A grown in DMEM produced 1800 units/10⁶ seeded cells/day, and when they were kept in JRH they produced 1100 units/10⁶ seeded cells/day. Again a clear difference was observed in production levels between PER.C6 and PER.C6/E2A, although this might partly be due to temperature differences (see above). There was however a significant difference with PER.C6/E2A cells between the concentration in DMEM vs the concentration in JRH medium, although this effect was almost completely lost in PER.C6 cells.

EPO expression data obtained in this system are summarized in table 4. PER.C6 and derivatives thereof can be used for scaling up the DNA transfections system. According to Wurm and Bernard (1999) transfections on suspension cells can be performed at 1-10 liter set-ups in which yields of 1-10 mg/l (0.1-1 pg/cell/day) of recombinant protein have been obtained using electroporation. There is a need for a system in which this can be well controlled and yields might be higher, especially for screening of large numbers of proteins and toxic proteins that cannot be produced in a stable setting. With the Lipofectamine transfections on the best PER.C6 cells in the absence of serum, we reached 590 units/million cells/day (+/-5.9 pg/cell/day when 1 ELISA unit is approximately 10 ng EPO), while PER.C6/E2A reached 31 pg/cell/day (in the presence of serum). The medium used for suspension cultures of PER.C6 and PER.C6/E2A (JRH ExCell 525) does not support efficient transient DNA transfections using components like PEI. Therefore the medium is adjusted to enable production of recombinant EPO after transfection of pEP02000/DHFRwt and pEP02000/DHFRm containing a recombinant human EPO cDNA, and pcDNA2000/DHFRwt containing other cDNA's encoding recombinant proteins.
1 to 10 liter suspension cultures of PER.C6 and PER.C6/E2A growing in adjusted medium to support transient DNA transfections using purified plasmid DNA, are used for electroporation or other methods, performing transfection with the same expression plasmids. After several hours the transfection medium is removed and replaced by fresh medium without serum. The recombinant protein is allowed to accumulate in the supernatant for several days after which the supernatant is harvested and all the cells are removed. The supernatant is used for down stream processing to purify the recombinant protein.

### Example 15: Purification and analysis of recombinant EPO.

Large batches of growing cells are produced in bioreactors, the secreted recombinant human EPO protein is purified according to procedures known by one of skill in the art. The purified recombinant human EPO protein from PER.C6 and PER.C6/E2A stable clones or transfectants is checked for glycosylation and folding by comparison with commercially available EPO and EPO purified from human origin (urine) using methods known to one of skill in the art (see example 16 and 17). Purified and glycosylated EPO proteins from PER.C6 and PER.C6/E2A are tested for biological activity in in vivo experiments and in mouse spleens as described (Krystal (1983) and in vitro assays (see example 18).

### Example 16: Activity of beta-galactoside alpha 2,6-sialyltransferase in PER.C6

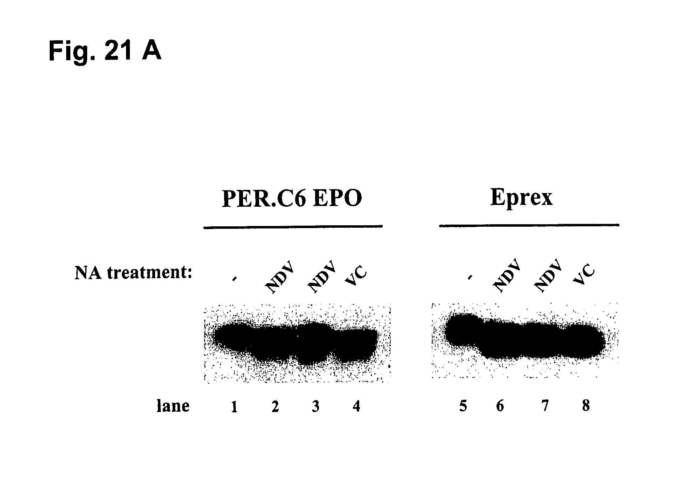

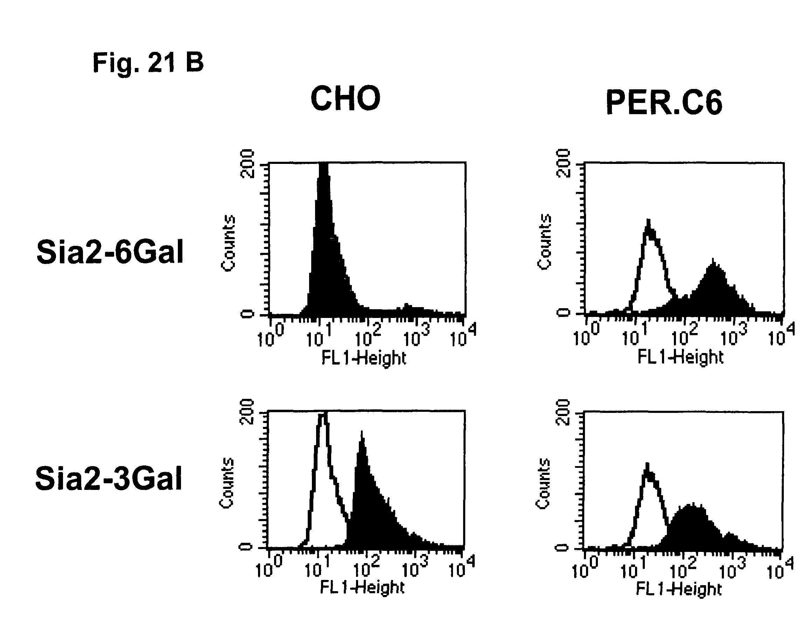

It is known that Chinese hamster ovary (CHO) cells do not contain a gene for beta-galactoside alpha 2,6-sialyltransferase, resulting in the absence of alpha 2,6-linked sialic acids at the terminal ends ofN- and O-linked oligosaccharides of endogenous and recombinant glycoproteins produced on these CHO cells. Since the alpha 2,3-sialyltransferase gene is present in CHO cells, proteins that are produced on these cells are typically from the 2,3 linkage type. EPO that was purified from human urine does however contain both alpha 2,3- and alpha 2,6-linked sialic acids. To determine whether PER.C6 cells, being a human cell line, are able to produce recombinant EPO containing both alpha 2,3- and alpha 2,6-linkages, a direct neuraminidase assay was performed on recombinant EPO produced on PER.C6 cells after transfection with EPO expression vectors. As a control, commercially available Eprex samples were used, which was derived from CHO cells and which should only contain sialic acid linkages of the alpha 2,3 type. The neuraminidases that were used were from Newcastle Disease Virus (NDV) that specifically cleaves alpha 2,3 linked neuraminic acids (sialic acids) from N- and O-linked glycans, and from Vibro cholerae (VC) that non-specifically cleaves all terminal N- or O-linked sialic acids (alpha 2,3, alpha 2,6 and alpha 2,8 linkages). Both neuraminidases were from Boehringer and were incubated with the samples according to guidelines provided by the manufacturer. Results are shown in Fig 21A. In lane 2 and 3 (treatment with NDV neuraminidase) a slight shift is observed as compared to lane 1 (non treated PER.C6 EPO). When this EPO sample was incubated with VC derived neuraminidase, an even faster migrating band is observed as compared to NDV treated samples. However, with the commercially available Eprex only a shift was observed when NDV derived neuraminidase was applied (lane 6 and 7 compared to non treated sample in lane 5) and not when VC neuraminidase was used (lane 8).
To further show that indeed no sialic acids of the alpha 2,6 linkage type are present on CHO cells, but that they do indeed exist in proteins present on the cell surface of PER.C6 cells, the following experiment was executed: CHO cells were released from the solid support using trypsin-EDTA, while for PER.C6, suspension cells were used. Both suspensions were washed once with Mem-5% FBS and incubated in this medium for 1 h at 37°C. After washing with PBS, the cells were resuspended to approximately 10⁶ cells/ml in binding medium (Tris-buffered saline, pH 7.5, 0.5%BSA, and 1mM each of Mg Cl₂, MnCl₂ and CaCl₂). Aliquots of the cells were incubated for 1 h at room temperature with DIG-labeled lectins, Sambucus nigra agglutinin (SNA) and Maackia amurensis agglutinin (MAA), which specifically bind to sialic acid linkages of the alpha 2,6 Gal and alpha 2,3 Gal types, respectively. Control cells were incubated without lectins. After I hour, both lectin-treated and control cells were washed with PBS and then incubated for 1 hour at room temperature with FITC-labeled anti-DIG antibody (Boehringer-Mannheim). Subsequently, the cells were washed with PBS and analyzed for fluorescence intensity on a FACsort apparatus (Becton Dickinson). The FACS analysis is shown in Fig 21B. When the SNA lectin is incubated with CHO cells no shift is seen as compared to non-treated cells, while when this lectin is incubated with PER.C6 cells, a clear shift (dark fields) is observed as compared to non-treated cells (open fields). When both cell lines are incubated with the MAA lectin both cell lines give a clear shift as compared to non-treated cells. From these EPO digestions and FACS results it is concluded that there is a beta-galactoside alpha 2,6 sialyltransferase activity present in human PER.C6 cells that is absent in hamster CHO cells.

### Example 17: Determination of sialic acid content in PER.C6 produced EPO

The terminal neuraminic acids (or sialic acids) that are present on the N- and O-linked glycans of EPO protect the protein from clearance from the bloodstream by enzymes in the liver. Moreover, since these sialic acids are negatively charged, one can distinguish between different EPO forms depending on their charge or specific pI. therefore, EPO produced on PER.C6 and CHO cells was used in 2-dimensional electrophoresis in which the first dimension separates the protein on charge (pH range 3-10) and the second dimension separates the proteins further on molecular weight. Subsequently, the proteins were blotted and detected in a western blot with an anti-EPO antibody.
It is also possible to detect the seperated EPO protein by staining the gel using Coomassie blue or silverstaining methods and subsequently removing different spots from the gel and determine the specific glycan composition of the different N- or O-linked glycosylations that are present on the protein by mass spectrometry.

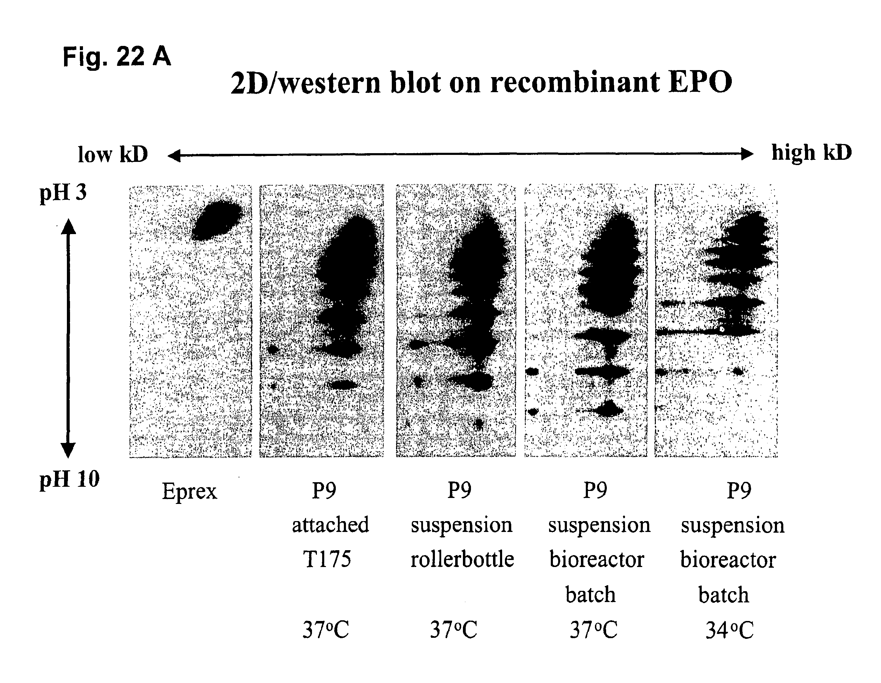

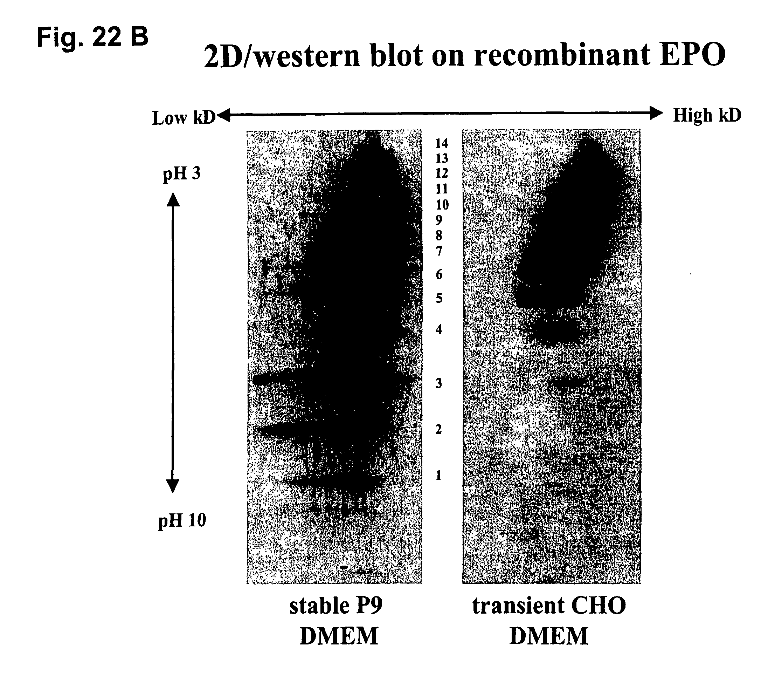

In Fig 22A a number of EPO samples are shown that were derived from P9 supernatants. P9 is the PER.C6 cell line that stably expresses recombinant human EPO (see example 8). These samples were compared to commercially available Eprex, which contains only EPO forms harboring approximately 9 to 14 sialic acids. Eprex should therefore be negatively charged and be focusing towards the pH 3 side of the gel. Fig 22B shows a comparison between EPO derived from P9 in an attached setting in which the cells were cultured on DMEM medium and EPO derived from CHO cells that were transiently transfected with the pEPO2000/DHFRwt vector. Apparently the lower forms of EPO can not be detected in the CHO samples whereas all forms can be seen in the P9 sample. The sialic acid content is given by numbering the bands that were seperated in the first dimension from 1 to 14. Since the western blot was performed by using ECL and since it is relatively unclear whether the antibody that was used to detect the EPO molecules on the blot and since it is unknown whether glycosylation can inhibit recognition by the antibody or transfer to the nitrocellulose, it is not possible to determine the percentage of each form that is present in these mixtures. However, it is possible to determine the presence of the seperate forms of sialic acid containing EPO molecules. It can be concluded that PER.C6 is able to produce the entire range of 14 sialic acid containing isoforms of recombinant human EPO.

### Example 18: In vitro functionality of PER.C6 produced EPO

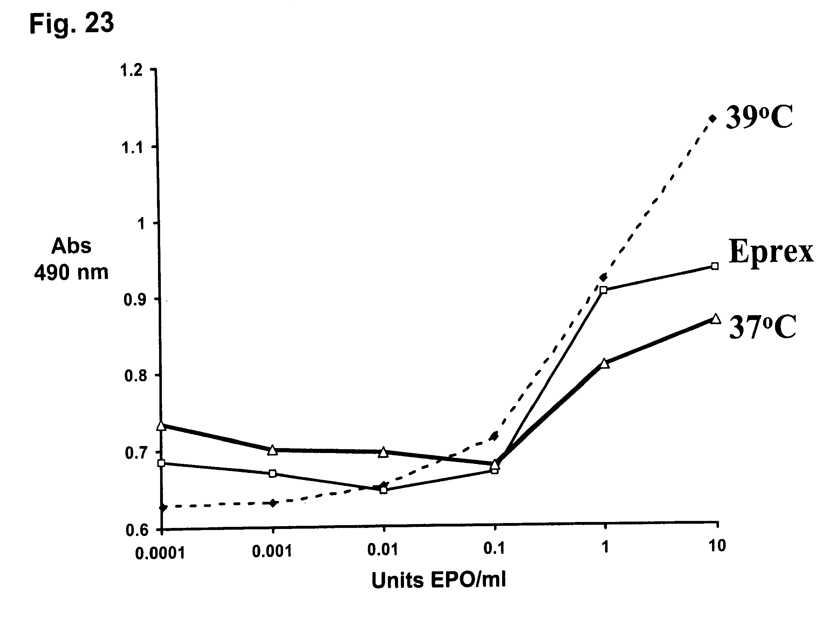

The function of recombinant EPO in vivo is determined by its half life in the bloodstream. Removal of EPO takes place by liver enzymes that bind to galactose residues in the glycans that are not protected by sialic acids and by removal through the kidney. Whether this filtering by the kidney is due to misfolding or due to under- or mis-glycosylation is unknown. Furthermore, EPO molecules that reach their targets in the bone marrow and bind to the EPO receptor on progenitor cells are also removed from circulating. Binding to the EPO receptor and downstream signalling depends heavily on a proper glycosylation status of the EPO molecule. Sialic acids can to some extent inhibit binding of EPO to the EPO receptor resulting in a lower effectivity of the protein. However, since the sialic acids prevent EPO from removal, these sugars are essential for its function to protect the protein on its travel to the EPO receptor. When sialic acids are removed from EPO in vitro, a better binding to the receptor occurs, resulting in a stronger downstream signalling. This means that the functionalities in vivo and in vitro are significantly different, although a proper EPO receptor binding property can be checked in vitro despite the possibility of an under-sialylation causing a short half life in vivo (Takeuchi et al. 1989).
Several in vitro assays for EPO functionality have been described of which the stimulation of the IL3, GM-CSF and EPO-dependent human cell line TF-1 is most commonly used. Hereby, one can determine the number of in vitro units per mg (Kitamura et al. 1989; Hammerling et al. 1996). Other in vitro assays are the formation of red colonies under an agarose layer of bone marrow cells that were stimulated to differentiate by EPO, the incorporation of ⁵⁹Fe into heme in cultured mouse bone marrow cells (Krystal et al. 1981 and 1983; Takeuchi et al. 1989), in rat bone marrow cells (Goldwasser et al. 1975) and the Radio Immuno Assay (RIA) in which the recognition of EPO for antisera is determined. EPO produced on PER.C6/E2A cells was used to stimulate TF-1 cells as follows: Cells were seeded in 96-wells plates with a density of around 10,000 cells per well in medium lacking IL3 or GM-CSF, which are the growth factors that can stimulate indefenite growth of these cells in culture. Subsequently, medium is added resulting in final concentrations of 0.0001, 0.001, 0.01, 0.1, 1 and 10 units per ml. These units were determined by ELISA, while the units of the positive control Eprex were known (4000 units per ml) and were diluted to the same concentration. Cells were incubated with these EPO samples for 4 days, after which an MTS assay (Promega) was performed to check for viable cells by fluorescence measurement at 490 nm (fluorescence is detectable after transfer of MTS into formazan). Figure 23 shows the activity of two samples derived from PER.C6/E2A cells that were transfected with an EPO expression vector and subsequently incubated at 37°C and 39°C for 4 days. The results suggest that samples obtained at 39°C are more active than samples obtained at 37°C which might indicate that the sialic acid content is suboptimal at higher temperatures. It is hereby shown that PER.C6 produced EPO can stimulate TF-1 cells in an in vitro assay, strongly suggesting that the EPO that is produced on this human cell line can interact with the EPO receptor and stimulate differentiation.

### Example 19: production of recombinant murine and humanized and human monoclonal antibodies in PER.C6 and PER.C6/E2A.

### A. Transient DNA transfections

cDNA's encoding heavy and light chains of murine, humanized and human monoclonal antibodies (mAbs) are cloned in two different systems: One in which the heavy and light chains are integrated into one single plasmid (a modified pcDNA2000/DHFRwt plasmid) and the other is a system in which heavy and light chain cDNA's are cloned separately into two different plasmids (see example 1, 3, 4 and 5). These plasmids can carry the same selection marker (like DHFR) or they carry each their own selection marker (one that contains the DHFR gene and one that contains for instance the neo-resistance marker). For transient expression systems it does not matter what selection markers are present in the backbone of the vector since no subsequent selection is being performed. In the common and regular transfection methods used in the art, equal amounts of plasmids are transfected. A disadvantage of integrating both heavy and light chain on one single plasmid is that the promoters that are driving the expression of both cDNA's might influence each other, resulting in non-equal expression levels of both subunits, although the number of cDNA copies of each gene is exactly the same.
Plasmids containing the cDNA's of the heavy and light chain of a murine and a humanized monoclonal antibody are transfected and after several days the concentration of correctly folded antibody is determined using methods known to persons skilled in the art. Conditions such as temperature and used medium are checked for both PER.C6 and PER.C6/E2A cells. Functionality of the produced recombinant antibody is controlled by determination of affinity for the specified antigen

### C. Stable production and amplification of the integrated plasmid

Expression plasmids carrying the heavy and light chain together and plasmids carrying the heavy and light chain separately are used to transfect attached PER.C6 and PER.C6/E2A and CHO-dhfr cells. Subsequently cells are exposed to MTX and/or hygromycin and neomycin to select for integration of the different plasmids. Moreover, a double selection with G418 and hygromycin is performed to select for integration of plasmids that carry the neomycin and hygromycin resistance gene. Expression of functional full length monoclonal antibodies is determined and best expressing clones are used for subsequent studies including stability of integration, copy number detection, determination of levels of both subunits and ability to amplify upon increase of MTX concentration after the best performing cell lines are used for mAb production in larger settings like perfused, and (fed-) batch bioreactors after which optimization of quantity and quality of the mAbs is executed.

### Example 20: Transfection of mAb expression vectors to obtain stable cell lines

PER.C6 cells were seeded in DMEM plus 10% FBS in 47 tissue culture dishes (10 cm diameter) with approximately 2.5 x 10⁶ cells per dish and were kept overnight under their normal culture conditions (10% CO₂ concentration and 37°C). The next day, co-transfections were performed in 39 dishes at 37°C using Lipofectamine in standard protocols with 1 ug MunI digested and purified pUBS-Heavy2000/Hyg(-) and 1 ug ScaI digested and purified pUBS-Light2001/Neo (see example 3) per dish, while 2 dishes were co-transfected as controls with 1 ug MunI digested and purified pcDNA2000/Hyg(-) and 1 ug ScaI digested and purified pcDNA2001/Neo. As a control for transfection efficiency, 4 dishes were transfected with a LacZ control vector, while 2 dishes were not transfected and served as negative controls.
After 5 hours, cells were washed twice with DMEM and refed with fresh medium without selection. The next day, medium was replaced by fresh medium containing different selection reagents: 33 dishes of the heavy and light chain co-transfectants, 2 dishes that were transfected with the empty vectors and the 2 negative controls (no transfection) were incubated only with 50 ug per ml hygromycin, 2 dishes of the heavy and light chain co-transfectants and 2 dishes of the transfection efficiency dishes (LacZ vector) were incubated only with 500 ug per ml G418, while 2 transfection efficiency dishes were not treated with selection medium but used for determining transfection efficiency that was around 40%. 2 dishes were incubated with a combination of 50 ug per ml hygromycin and 250 ug per ml G418 and 2 dishes were incubated with 25 ug per ml hygromycin and 500 ug per ml G418.

Since cells were overgrowing when they were only incubated with hygromycin alone it was decided that a combination of hygromycin and G418 selection would immediately kill the cells that integrated only one type of the two vectors that were transfected. So, 7 days after seeding, all co-transfectants were further incubated with a combination of 100 ug per ml hygromycin and 500 ug per ml G418. Cells were refreshed 2 or 3 days with medium containing the same concentrations of selecting agents. 14 days after seeding the concentrations were adjusted to 250 ug per ml G418 and 50 ug per ml hygromycin. 22 days after seeding a large number of colonies had grown to an extent in which it was possible to select, pick and subculture. Approximately 300 seperate colonies were selected and picked from the 10 cm dishes and subsequently grown via 96-wells and/or 24-wells via 6-wells plates to T25 flasks. In this stage, cells are frozen (4 vials per subcultured colony) and production levels of recombinant UBS-54 mAb are determined in the supernatant using the ELISA described in example 26.
CHO-dhfr cells are seeded in DMEM plus 10% FBS and including hypoxanthine and thymidine in tissue culture dishes (10 cm diameter) with approximately 1 million cells per dish and are kept overnight under normal conditions and used for a co-transfection the next day with pUBS-Heavy2000/Hyg(-) and pUBS-Light2001/DHFRwt under standard protocols using Lipofectamine. Medium is replaced with fresh medium after a few hours and split to different densities to allow the cells to adjust to the selection medium when stable integration is taking place without a possible outgrowth of non-transfected cells. Colonies are first selected on hygromycin resistance and subsequently MTX is added to select for double integrations of the 2 plasmids in these subcultured cell lines. Transfections as described for pUBS-Heavy2000/Hyg(-) and pUBS-Light2001/Neo are performed with pUBS2-Heavy2000/Hyg(-) and pUBS2-Light2001/Neo in PER.C6 and PER.C6/E2A and selection is performed with either subsequent incubation with hygromycin followed by G418 or as described above with a combination of both selection reagents. CHO-dhfr cells are transfected with pUBS2-Heavy2000/Hyg(-) and pUBS2-Light2001/DHFRwt as described for pUBS-Heavy2000/Hyg(-) and pUBS-Light2001/DHFRwt and selection is performed in a sequential way in which cells are first selected with hygromycin after which an integration of the light chain vector is controlled for by selection on MTX.

Furthermore, PER.C6 and PER.C6/E2A cells are also used for transfections with pUBS-3000/Hyg(-) and pUBS2-3000/Hyg(-), while CHO-dhfr cells are transfected with pUBS-3000/DHFRwt and pUBS2-3000/DHFRwt after which a selection and further amplification of the integrated plasmids is performed by increasing the MTX concentration. In the case of the pcDNAs3000 plasmids, an equal number of mRNA's of both heavy and light chain is expected, while in the case of two seperate vectors it is unclear whether a correct equilibrium is achieved between the two subunits of the immunoglobulin.
Transfections are also being performed on PER.C6, PER.C6/E2A and CHO-dhfr with expression vectors described in example 4 and 5 to obtain stable cell lines that express the humanized IgG1 mAb CAMPATH-1H and the humanized IgG1 mAb 15C5 respectively.

### Example 21: Sub-culturing of transfected cells.

From PER.C6 cells transfected with pUBS-Heavy2000/Hyg (-) and pUBS-light2001/Neo, approximately 300 colonies that were growing in medium containing Hygromycin and G418 were generally grown subsequently in 96-wells, 24-wells and 6-wells plates in their respective medium plus their respective selecting agents. Cells that were able to grow in 24 wells plates, were checked for mAb production by using the ELISA described in example 26. If cells scored positively, at least one vial of each clone was frozen and stored, and cells were subsequently tested and subcultured. The selection of a good producer clone is based on high expression, culturing behavior and viability. To allow checks for long term viability, amplification of the integrated plasmids and suspension growth during extended time periods, best producer clones are frozen, of which a number of the best producers of each cell line are selected for further work. Similar experiments are being performed on CHO-dhfr cells transfected with different plasmids and PER.C6 and PER.C6/E2A cells that were transfected with other combinations of heavy and light chains and other combinations of selection markers.

### Example 22: mAb production in bioreactors

The best UBS-54 producing transfected cell line of PER.C6 is brought into suspension by washing the cells in PBS and then culturing the cells in JRH ExCell 525 medium, first in small culture flasks and subsequently in rollerbottles, and scaled up to 1 to 2 liter fermentors. Cells are kept on hygromycin and G418 selection till it is proven that integration of the vectors is stable over longer periods of time. This is done when cells are still in their attached phase or when cells are in suspension.
Suspension growing mAb producing PER.C6 cells are generally cultured with hygromycin and G418 and used for inoculation of bioreactors from rollerbottles. Production yields, functionality and quality of the produced mAb is checked after growth of the cells in perfused bioreactors and in fed batch settings.

### A. Perfusion in a 2 liter bioreactor.

Cells are seeded in suspension medium in the absence of selecting agents at a concentration of approximately 0.5 x 10⁶ cells per ml and perfusion is started after a number of days when cell density reaches approximately 2 to 3 x 10⁶ cells per ml. The perfusion rate is generally 1 volume per 24 hours with a bleed of approximately 250 ml per 24 hours. In this setting, cells stay normally at a constant density of approximately 5 x 10⁶ cells per ml and a viability of almost 95% for over a month. The mAb production levels are determined on a regular basis.

### B. Fed batch in a 2 liter bioreactor.

In an initial run, mAb producing PER.C6 suspension cells that are grown on rollerbottles are used to inoculate a 2 liter bioreactor in the absence of selecting agents to a density of 0.3 to 0.5 million cells per ml in a working volume of 300 to 500 ml and are left to grow till the viability of the cell culture drops to 10%. As a culture lifetime standard it is determined at what day after inoculation, the viable cell density drops beneath 0.5 million cells per ml. Cells normally grow till a density of 2 to 3 million cells per ml after which the medium components become limiting and the viability decreases. Furthermore, it is determined how much of the essential components, like glucose and amino acids in the medium are being consumed by the cells. Next to that, it is determined what amino acids are being produced and what other products are accumulating in the culture. Depending on this, concentrated feeding samples are being produced that are added at regular time points to increase the culture lifetime and thereby increase the concentration of the mAb in the supernatant. In another setting 10x concentrated medium samples are developed that are added to the cells at different timepoints and that also increase the viability of the cells for a longer period of time, finally resulting in a higher concentration of mab in the supernatant.

### Example 23: Transient expression of humanized recombinant monoclonal antibodies.

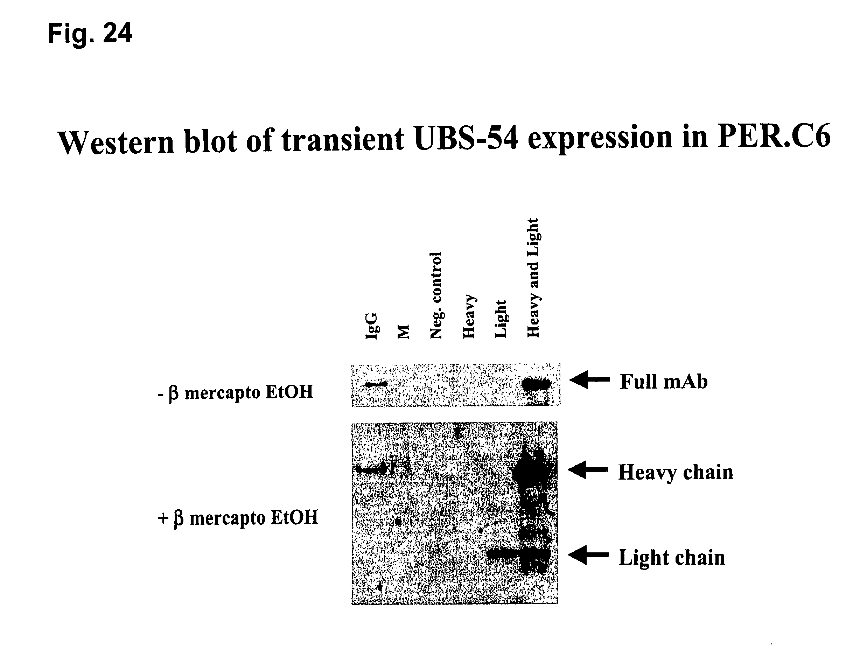

The correct combinations of the UBS-54 heavy and light chain genes containing vectors were used in transient transfection experiments in PER.C6 cells. For this, it is not important which selection marker is introduced in the plasmid backbone, because the expression lasts for a short period (2-3 days). The transfection method is generally Lipofectamine although other cationic lipid compounds for efficient transfection can be used. Transient methods are extrapolated from T25 flasks settings to at least 10-liter bioreactors. Approximately 3.5 million PER.C6 and PER.C6/E2A cells were seeded at day 1 in a T25 flask. At day 2, cells were transfected with, at most, 8 ug plasmid DNA using Lipofectamine and refreshed after a 2-4 hours and left for 2 days. Then, the supernatant was harvested and antibody titers were measured in a quantitative ELISA for human IgG1 immunoglobulins (CLB, see also example 26). Levels of total human antibody in this system are approximately 4.8 ug/million seeded cells for PER.C6 and 11.1 ug/million seeded cells for PER.C6/E2A. To determine how much of the produced antibody is of full size and built up from two heavy and two light chains, as well as the expression levels of the heavy and/or light chain alone and connected by disulfide bridges, control ELISA's recognizing the sub-units separately are developed. Different capturing and staining antibody combinations are used that all detect human(ized) IgG1 sub-units. Supernatants of PER.C6 transfectants (transfected with control vectors or pUBS-Heavy2000/Hyg(-) and pUBS-Light2001/DHFRwt) were checked for full sized mAb production (Fig 24). Samples were treated with and without DTT, whereby one can distinguish between full sized mAb (non reduced) and heavy and light chain separately (reduced). As expected, the heavy chain is only secreted when the light chain is co-expressed and most of the antibody is of full size.

### Example 24: Scale-up system for transient transfections

PER.C6 and derivatives thereof are used for scaling up the DNA transfections system. According to Wurm and Bernard (1999), transfections on suspension cells can be performed at 1-10 liter set-ups in which yields of 1-10 mg/l (0.1-1 pg/cell/day) of recombinant protein have been obtained using electroporation.
There is a need for a system in which this can be well controlled and yields might be higher, especially for screening of large numbers of proteins and toxic proteins that cannot be produced in a stable setting. Moreover, since cell lines like CHO are heavily affected by apoptosis-inducing agents like lipofectamine, the art teaches that there is a need for cells that are resistant to this. Since PER.C6 is hardly affected by transfection methods it seems that PER.C6 and derivatives thereof are useful for these purposes. 1 to 50 liter suspension cultures of PER.C6 and PER.C6/E2A growing in adjusted medium to support transient DNA transfections using purified plasmid DNA, are used for electroporation or other methods, performing transfection with the same expression plasmids. After several hours the transfection medium is removed and replaced by fresh medium without serum. The recombinant protein is allowed to accumulate in the supernatant for several days after which the supernatant is harvested and all the cells are removed. The supernatant is used for down stream processing to purify the recombinant protein

### Example 26: Development of an ELISA for determination of human mAbs

Greiner microlon plates # 655061 were coated with an anti-human IgG1 kappa monoclonal antibody (Pharmingen #M032196 0.5) with 100 ul per well in a concentration of 4 ug per ml in PBS. Incubation was performed overnight at 4°C or for 90 minutes at 37°C. Then, wells were washed three times with 0.05% Tween/PBS (400 ul per well) and subsequently blocked with 100 ul 5% milk dissolved in 0.05% Tween/PBS per well for 30 minutes at 37°C and then, plate was washed three times with 400 ul 0.05% Tween/PBS per well. As a standard, a purified human IgG1 antibody was used (Sigma, #108H9265) diluted in 0.5% milk/0.05% Tween/PBS in dilutions ranging from 50 to 400 ng per ml. Per well 100 ul standard was incubated for 1 h at 37°C. Then, the plate was washed three times with 400 ul per well 0.05% Tween/PBS. As the second antibody a biotin labelled mouse monoclonal anti-human IgG1 antibody was used (Pharmingen #M045741) in a concentration of 2 ng per ml. Per well 100 ul of this antibody was added and incubated for 1 h at 37°C and the wells were washed three times with 400 ul 0.05% Tween/PBS. Subsequently, conjugate was added: 100 ul per well of a 1:1000 dilution of Streptavidin-HRP solution (Pharmingen #M045975) and incubated for 1 h at 37°C, and the plate was again washed three times with 400 ul per well with 0.05% Tween/PBS. One ABTS tablet (Boehringer Mannheim #600191-01) was dissolved in 50 ml ABTS buffer (Boehringer Mannheim #60328501) and 100 ul of this solution was added to each well and incubated for 1 h at RT or 37°C. Finally, the OD was measured at 405 nm. Supernatant samples from cells transfected with mAb encoding vectors, were generally dissolved and diluted in 0.5% milk/0.05% Tween/PBS. If samples did not fit with the linear range of the standard curve, other dilutions were used.

### Example 27: Production of Influenza HA and NA proteins in a human cell for recombinant subunit vaccines

cDNA sequences of genes encoding haemagluttinine (HA) and neuraminidase (NA) proteins of known and regularly appearing novel Influenza virus strains are being determined and generated by PCR with primers for convenient cloning into pcDNA2000, pcDNA2001, pcDNA2002 and pcDNAs3000 vectors (see example 1). Subsequently, these resulting expression vectors are being transfected into PER.C6 and derivatives thereof for stable and transient expression of the recombinant proteins to result in the production of recombinant HA and NA proteins that are therefore produced in a complete standardized way with human cells under strict and well defined conditions. Cells are let to accumulate these recombinant HA and NA recombinant proteins for a standard period of time. When the pcDNAs3000 vector is used, it is possible to clone both cDNA's simultaneously and have the cells produce both proteins at the same time. From seperate or combined cultures, the proteins are being purified following standard techniques and final HA and NA titers are being determined by persons skilled in the art and activities of the proteins are checked. Then, the purified recombinant proteins are used for vaccination studies and finally used for large scale vaccination purposes.

The HA1 fragment of the swine influenza virus A/swine/Oedenrode/7C/96 (Genbank accession number AF092053) was Obtained by PCR using a forward primer with the following sequence (SEQ. ID. NO. 27): 5' ATT GGC GCG CCA CCA TGA AGA CTA TCA TTG CTT TGA GCT AC 3', and with a reverse primer with the following sequence (SEQ. ID. NO. 28): 5' GAT GCT AGC TCA TCT AGT TTG TTT TTC TGG TAT ATT CCG 3'. The resulting 1.0 kb containing PCR product was digested with AscI and NheI restriction enzymes and ligated with a AscI and NheI digested and purified pcDNA2001/DHFRwt vector, resulting in pcDNA2001/DHFRwt-swHA1. Moreover, the HA2 fragment of the same virus was amplified by PCR using the same forward primer as described for HA1 and another reverse primer with the following sequence (SEQ. ID. NO. 29): 5' GAT GCT AGC TCA GTC TTT GTA TCC TGA CTT CAG TTC AAC ACC 3'. The resulting 1.6 kb HA2 PCR product was cloned in an identical way as described for HA1, resulting in pcDNA2001/DHFRwt-swHA2.

### Example 28: Integration of cDNA's encoding post-transational modifying enzymes.

Since the levels of recombinant protein production in stable and transiently transfected and infected PER.C6 and PER.C6/E2A are extremely high and since a higher expression level is usually obtained upon DHFR dependent amplification due to increase of MTX concentration, an "out-titration" of the endogenous levels of enzymes that are involved in post-translational modifications might occur.
Therefore, cDNA's encoding human enzymes involved in different kinds of post-translational modifications and processes like glycosylation, phosphorylation, carboxylation, folding and trafficking are being overexpressed in PER.C6 and PER.C6/E2A to enable a more functional recombinant product to be produced to extreme levels in small and large settings. It was shown that CHO cells can be engineered in which an alpha-2,6-sialyltransferase was introduced to enhance the expression and bioactivity of tPA and human erythropoietin (Zhang et al. 1998, Minch et al. 1995, Jenkins et al 1998). Other genes like beta 1,4-galactosyltransferase were also introduced into insect and CHO cells to improve the N-linked oligosaccharide branch structures and to enhance the concentration of sialic acids at the terminal residues (Weikert et al. 1999; Hollister et al 1998). PER.C6 cells are modified by integration of cDNA's encoding alpha 2,3-sialyltransferase, alpha 2,6-sialyltransferase and beta 1,4 galactosyltransferase proteins to further increase the sialic acid content of recombinant proteins produced on this human cell line.

### Example 31: Generation of PER.C6 derived cell lines lacking a functional DHFR protein

PER.C6 cells are used to knock out the DHFR gene using different systems to obtain cell lines that can be used for amplification of the exogenous integrated DHFR gene that is encoded on the vectors that are described in examples 1 to 5 or other DHFR expressing vectors. PER.C6 cells are screened for the presence of the different chromosomes and are selected for a low copynumber of the chromosome that carries the human DHFR gene. Subsequently, these cells are used in knock-out experiments in which the open reading frame of the DHFR gene is disrupted and replaced by a selection marker. To obtain a double knock-out cell line, both alleles are removed via homologous recombination using two different selection markers or by a other systems as for instance described for CHO cells (Urlaub et al. 1983).
Other systems are also applied in which the functionality of the DHFR protein is lowered or completely removed, for instance by the use of anti-sense RNA or via RNA/DNA hybrids, in which the gene is not removed or knocked out, but the downstream products of the gene are disturbed in their function.

### Example 32: Long term production of recombinant proteins using protease and neuraminidase inhibitors.

Stable clones described in example 8 are used for long term expression in the presence and absence of MTX, serum and protease inhibitors. When stable or transfected cells are left during a number of days to accumulate recombinant human EPO protein, a flattening curve instead of a straight increase is observed which indicates that the accumulated EPO is degraded in time. This might be an inactive process due to external factors like light or temperature. It might also be that specific proteases that are produced by the viable cells or that are released upon lysis of dead cells, digest the recombinant EPO protein. Therefore an increasing concentration of CuSO₄ is added to the culture medium after transfection and on the stable producing cells to detect a more stable production curve: Cells are cultured for several days and the amount of EPO is determined at different timepoints. CuSO₄ is a known inhibitor of protease activity, which can be easily removed during down stream processing and EPO purification. The most optimal concentration of CuSO₄ is used to produce recombinant human EPO protein after transient expression upon DNA transfection and viral infections. Furthermore, the optimal concentration of CuSO₄ is also used in the production of EPO on the stable clones. In the case of EPO in which the presence of terminal sialic acids are important to ensure a long circulation half life of the recombinant protein, it is necessary to produce highly sialylated EPO. Since living cells produce neuraminidases that can be secreted upon activation by stress factors, it is likely that produced EPO loose their sialic acids due to these stress factors and produced neuraminidases. To prevent clipping off of sialic acids, neuraminidase inhibitors are added to the medium to result in a prolonged attachment of sialic acids to the EPO that is produced.

### Example 33: Stable expression of recombinant proteins in human cells using the amplifiable Glutamine Synthetase system

PER.C6 and derivatives thereof are being used to stably express recombinant proteins using the glutamine synthetase (GS) system. First, cells are being checked for their ability to grow in glutamine free medium. If cells can not grow in glutamine free medium this means that these cells do not express enough GS, finally resulting in death of the cells. The GS gene can be integrated into expression vectors as a selection marker (as is described for the DHFR gene) and can be amplified by increasing the methionine sulphoximine (MSX) concentration resulting in overexpression of the recombinant protein of interest, since the entire stably integrated vector will be co-amplified as was shown for DHFR. The GS gene expression system became feasible after a report of Sanders et al. (1984) and a comparison was made between the DHFR selection system and GS by Cockett et al. (1990). The production of recombinant mAbs using GS was first described by Bebbington et al. (1992).
The GS gene is cloned into the vector backbones described in example 1 or cDNA's encoding recombinant proteins and heavy and light chains of mAbs are cloned into the available vectors carrying the GS gene. Subsequently, these vectors are transfected into PER.C6 and selected under MSX concentrations that will allow growth of cells with stable integration of the vectors.

### Example 34: Production of recombinant HIV gp 120 protein in a human cell

The cDNA encoding the highly glycosylated envelope protein gp120 from Human Immunodeficiency Virus (HIV) is determined and obtained by PCR using primers that harbor a perfect Kozak sequence in the upstream primer for proper translation initiation and conventient restriction recognition sequences for cloning into the expression vectors described in example 1. Subsequently, this PCR product is sequenced on both strands to ensure that no PCR mistakes are being introduced.
The expression vector is transfected into PER.C6, derivatives thereof and CHO-dhfr cells to obtain stable producing cell lines. Differences in glycosylation between CHO-produced and PER.C6 produced gp120 are being determined in 2D electrophoresis experiments and subsequently in Mass Spectrometry experiments, since gp120 is a heavily glycosylated protein with mainly O-linked oligosaccharides. The recombinant protein is purified by persons skilled in the art and subsequently used for functionality and other assays. Purified protein is used for vaccination purposes to prevent HIV infections.

### Figure legends

1. pcDNA2000/DHFRwt
2. pcDNA2001/DHFRwt
3. pcDNA2002/DHFRwt
4. pcDNAs3000/DHFRwt
5. pEPO2000/DHFRwt
7. pHC2000/Hyg(-)
8. pLC2001/DHFRwt
9. pUBS-Heavy2000/Hyg(-)
10. pUBS-Light2001/DHFRwt
11. pUBS2-Heavy2000/Hyg(-)
12. pUBS2-Light2001/DHFRwt
13. pUBS-3000/DHFRwt
14. pUBS2-3000/DHFRwt
15. EPO concentration in a 2 liter perfusion bioreactor given in ELISA units per ml. 6. EPO concentration in a 2 liter repeated batch bioreactor in which cell densities were brought back to 0.5x10⁶ cells per ml every 2 to 3 days.
16. EPO concentration from stable producing P9 cells, three days after inoculation with 0.3x10⁶ cells per ml in a 4x1 liter repeated batch bioreactor setting with different conditions: Left. Different settings for Dissolved Oxygen. Middle. Different temperatures. Right. Different constant pH. Standard settings for P9 cells are 50% DO, 37°C and pH 7.3. In every run a separate control run with these settings was performed depicted as the fourth bar in each set.
17. EPO production upon increase in MTX concentration in PER.C6 derived cell lines P8 and P9 calculated as ELISA units per million seeded cells per day.
18. Amplification of the DHFR gene upon increase in MTX concentration. Southern blot of BgIII digested DNA derived from normal PER.C6 cells (not grown in the presence of MTX) and from P8 and P9 cells (cultured in the presence of 100 nM, 800 nM and 1800 nM MTX). The blot was first hybridized with a radioactive DHFR probe, subsequently stripped and then incubated with a radioactive adenovirus E1 probe as an internal control.
19. Stability of recombinant EPO expression of two cell lines: P8(A) and P9(B). Cells were cultured in the presence or absence of MTX for approximately 60 passages in a time period of more than 4 months and EPO production was calculated as ELISA units per million seeded cells per day.
20. Activity of beta-galactoside alpha2,6-sialyltranferase in PER.C6 as compared to CHO cells. A. Western blot of EPO from supernatant of PER.C6 cells that were transfected with an EPO expression plasmid (left) and Eprex (right). PER.C6-EPO was run without treatment (lane 1), after treatment with NDV derived neuraminidase in two separate buffers (lanes 2 and 3) and after treatment with VC derived neuraminidase (lane 4). Eprex was also run without treatment (lane 5) and after similar treatments with neuraminidases (lanes 5 to 8). B. Shift in FACS analysis of PER.C6 (right panels) and CHO cells (left panels) after treatment with an anti-DIG antibody recognizing two DIG-linked lectins that were incubated with the cells (resulting in the dark fields), that either specifically recognize alpha2,6-linkages between sialic acids and galactoses (Sambucus nigra agglutinin, upper panels) and alpha2,3 sialic acids-galactose linkages (Maackia amurensis agglutinin, lower panels) as compared to a FACS analysis with non-lectin treated cells (open fields).
21. 2D/western analysis of recombinant EPO produced in different culture settings. A. P9 cells, stably expressing recombinant human EPO were grown in T175 flasks in DMEM and in rollerbottles and bioreactors in JRH ExCell 525 medium, and separated on 2D electrophoresis, blotted and incubated with H162, an anti-EPO antiserum. Samples containing the entire range of sialic acid containing EPO (from P9 supernatants) were compared to commercially available Eprex that contains solely the higher sialic acid containing EPO forms (left). B. EPO derived from attached P9 cells grown in DMEM medium in an attached setting compared to EPO derived from transiently transfected CHO cells that were also cultured in DMEM. FBS was present during culturing, but was removed before EPO accumulation was initiated. The suggested sialic acid contents in these samples (1-14) are indicated between the two blots.
22. In vitro functionality assay of PER.C6/E2A-EPO produced at 37°C using human TF-1 cells as compared to commercially available Eprex which is produced on CHO cells.
23. Western blot using an anti-human IgG 1 heavy chain antibody on supernatants from PER.C6 cells that were co-transfected with pUBS-Heavy2000/Hyg(-) and pUBS-Light2001/DHFRwt (lane 6) or with the vectors separately (lanes 4 and 5). As a positive control diluted human serum was loaded (IgG, lane 1) and as negative controls an expression vector encoding LacZ was transfected (control, lane 3) and a marker (M, sizes not depicted)was loaded (lane 2). The upper panel is from a gel that was loaded with samples from the transfectants that were not treated with DTT in which the heavy and light chain disulfide bridges stay intact and a full mAb of approximately 220 kD is detected. The lower panel shows samples from the same transfectants treated with DTT in which the disulfide bridges are removed resulting in a complete separation of the heavy and light chain. The blot was incubated with an antibody that recognizes human IgG1 heavy chains. The light chain is stained due to background recognition by the second (polyclonal) antibody used for the ECL-western procedure.

### REFERENCES

Baldwin RW, Byers VS (1986) Monoclonal antibodies in cancer treatment. Lancet 1,603-605.
Barbas CF, Kang AS, Lerner RA and Benkovic SJ (1991) Assembly of combinatorial antibody libraries on phage surfaces: The gene III site. Proc Natl Acad Sci USA. 88, 7978
Bebbington CR, Renner G, Thomson S, Abrams D, Yarranton GT (1992) High-level expression of a recombinant antibody from myeloma cells using a glutamine synthetase gene as an amplifiable selectable marker. Bio/technology 10, 169-175.
Borrebaeck CAK, Malmborg A-C and Ohlin M (1993) Does endogenous glycosylation prevent the use of mouse monoclonal antibodies as cancer therapeutics? Immunology Today 14, 477-479.
Borrebaeck CAK (1999) Human monoclonal antibodies: The emperor's new clothes? Nature Biotech. 17, 621.
Boshart W, Weber F, Jahn G, Dorsch-Hasler K, Fleckenstein B, Schaffner W (1985) A very strong enhancer is located upstream of an immediate early gene of human cytomegalovirus. Cell 41, 521-530.
Bruggeman M, Spicer C, Buluwela L, Rosewell I, Barton S, Surani MA, Rabbits TH (1991) Human antibody production in transgenic mice: expression from 100 kb of the human IgH locus. Eur J Immunol. 21, 1323-1326.
Bulens F., Vandamme A-M., Bernar H., Nelles L., Lijnen RH. and Collen D (1991) Construction and characterization of a functional chimeric murine-human antibody directed against human fibrin fragment-D dimer. Eur J Biochem. 195, 235-242.
Burton DR and Barbas III CF (1994) Human antibodies from combinatorial libraries. Adv Immunol. 57, 191-280.
Carter P, Presta L, Gorman CM, Ridgway JB, Henner D, Wong WL, Rowland AM, Kotts C, Carver ME and Shephard HM (1992) Humanization of an anti-p185HER2 antibody for human cancer therapy. Proc Natl Acad Sci USA 89, 4285-4289.
Clarkson T, Hoogenboom HR, Griffiths A and Winter G (1991) Making antibody fragments using phage display libraries. Nature 353, 624.
Cockett MI, Bebbington CR, Yarranton GT (1990) High level expression of tissue inhibitor of metalloproteinases in chines hamster ovary cells using glutamine synthetase gene amplification. Bio/technology 8, 662-667.
Crowe JS., Hall VS., Smith MA., Cooper HJ. and Tite JP (1992) Humanized monoclonal antibody CAMPATH-1 H: myeloma cell expression of genomic constructs, nucleotide sequence of cONA constructs and comparison of effector mechanisms of myeloma and Chinese hamster ovary cell-derived material. Clin Exp Immunol 87, 105-110.
Debbas M, White E (1993) Wild-type p53 mediates apoptosis by E1A, which is inhibited by E1B. Genes Dev. 7, 546-554.
Delorme E, Lorenzini T, Giffin J, Martin F, Jacobsen F, Boone T, Elliot S (1992) Role of glycosylation on the secretion and biological activity of erythropoietin. Biochemistry 31, 9871-9876.
Farrow SN, White JH, Martinou I, Raven T, Pun KT, Grinham CJ, Martinou JC, Brown R (1995) Cloning of a bcl-2 homologue by interaction with adenovirus E1B 19K. Nature 374, 731-733.
Fishwild DM, O'Donnell SL, Bengoechea T, Hudson DV, Harding F, Bernhard SL, Jones D, Kay RM, Higgins KM, Schramm SR, Lonberg N (1996) High-avidity human IgG kappa monoclonal antibodies from a novel strain of minilocus transgenic mice. Nat Biotechnol. 14, 845-851.
Fussenegger M, Bailey JE, Hauser H, Mueller PP (1999) Genetic optimization of recombinant glycoprotein production by mammalian cells. Trends Biotechn. 17, 35-42.
Frödin JE, Lefvert AK, Mellstedt H (1990) Pharmacokinetics of the mouse monoclonal antibody 17-1 A in cancer patients receiving various treatment schedules. Cancer Res. 50, 4866-4871.
Galili U (1993) Interaction of the natural anti-Gal antibody with alpha-galactosyl epitopes: a major obstacle for xenotransplantation in humans. Immunol Today 14, 480-482.
Garrard L, Yang M, O'Connell M, Kelley R and Henner DJ (1991) Fab assembly and enrichment in a monovalent phage display system. BioTechnology 9, 1373.
Goldwasser E, Eliason JF, Sikkema D (1975) An assay for erythropoietin in vitro at the milliunit level. Endocrinology 97, 315-23.
Green LL, Hardy MC, Maynard-Currie CE, Tsuda H, Louie DM, Mendez MJ, Abderrahim H, Noguchi M, Smith DH, Zeng Y (1994) Antigen-specific human monoclonal antibodies from mice engineered with human Ig heavy and light chain YACs. Nat Genet. 7, 13-21.
Gorman CM, Gies D, McCray G, Huang M (1989) The human cytomegalovirus major immediate early promoter can be trans-activated by adenovirus early proteins. Virology 171, 377-385.
Hale G, Dyer MJS, Clark MR, Phillips JM, Marcus R, Reichmann L, Winter G and Waldmann H (1988) Remission induction in non-Hodgkin lymphoma with reshaped human monoclonal antibody CAMPATH-1H. Lancet 2, 1394-1399.
Hammerling U, Kroon R, Wilhelmsen T, Sjödin L (1996) In vitro bioassay for human erythropoietin based on proliferative stimulation of an erythroid cell line and analysis of carbohydrate-dependent microheterogeneity. J Pharm Biomed An. 14, 1455-1469.
Han J, Sabbatini P, Perez D, Rao L, Modha D, White E (1996) The E1B 19K protein blocks apoptosis by interacting with and inhibiting the p53-inducible and death-promoting Bax protein. Genes Dev. 10, 461-477.
Havenga MJ, Werner AB, Valerio D, van Es HH (1998) Methotrexate selectable retroviral vectors for Gaucher disease. Gene Ther. 5, 1379-1388.
Hollister JR, Shaper JH, Jarvis DJ (1998) Stable expression of mammalian betal,4-galactosyltransferase extends the N-glycosylation pathway in insect cells. Glycobiology 8, 473-480.
Huls GA., Heijnen IAFM., Cuomo ME., Koningsberger JC., Wiegman L., Boel E., Van der Vuurst de Vries A-R., Loyson SAJ., Helfrich W., Van Berge Henegouwen GP., Van Meijer M., De Kruif J. and Logtenberg T (1999) A recombinant, fully human monoclonal antibody with antitumor activity constructed from phage-displayed antibody fragments. Nature Biotechnol. 17,276-281.
Isaacs JD, Watts RA, Hazleman BL, Hale G, Keogan MT, Cobbold SP, Waldmann H (1992) Humanised monoclonal antibody therapy for rheumatoid arthritis. Lancet 340, 748-52.
Jacobovits A (1995) Production of fully human antibodies by transgenic mice. Curr Opin Biotechnol. 6, 561-566.
Jenkins N, Parekh RB, James DC (1996) Getting the glycosylation right: implications for the biotechnology industry. Nat Biotechnol. 14, 975-81.
Jenkins N, Buckberry L, Marc A, Monaco L (1998) Genetic engineering of alpha 2,6-sialyltransferase in recombinant CHO cells. Biochem Soc Trans. 26, S115.
Kawashima I, Ozawa H, Kotani M, Suzuki M, Kawano T, Gomibuchi M, Tai T (1993) Characterization of ganglioside expression in human melanoma cells: immunological and biochemical analysis. J Biochem (Tokyo) 114, 186-193.
Kay R, Takei F, Humphries RK (1990) Expression cloning of a cDNA encoding M1/69. J Immunol. 145, 1952-1959.
Kitamura T, Tange T, Terasawa T, Chiba S, Kuwaki T, Miyagawa K, Piao Y-F, Miyazono K, Urabe A, Takaku F (1989) Establishment and characterization of a unique human cell line that proliferates dependently on GM-CSF, IL-3, or erythropoietin. J Cell Physiol. 140, 323-334.
Köhler G and Milstein C (1975) Continuous cultures of fused cells secreting antibody of predefined specificity. Nature 256:495.
Krystal, G. Eaves, AC, Eaves CJ (1981) A quantitative bioassay for erythropoietin, using mouse bone marrow. J Lab Clin Med. 97, 144-157.
Krystal G (1983) A simple microassay for erythropoietin based on 3H-thymidine incorporation into spleen cells from phenylhydrazine treated mice. Exp Hematol. 11, 649-660.
Lee EU, Roth J, Paulson JC (1989) Alteration of terminal glycosylation sequences on N-linked oligosaccharides of Chinese hamster ovary cells by expression of beta-galactoside alpha 2,6-sialyltransferase. J Biol Chem. 264, 13848-13855.
Levrero M, Barban V, Manteca S, Ballay A, Balsamo C, Avantaggiata ML, Natoli G, Skellekens H, Tiollais P, Perricaudet M (1991) Defective and non-defective adenovirus vectors for expression foreign genes in vitro and in vivo. Gene 101, 195-202.
Lonberg N, Taylor LD, Harding FA, Trounstine M, Higgins KM, Schramm SR, Kuo CC, Mashayekh R, Wymore K, McCabe JG (1994) Antigen-specific human antibodies from mice comprising four distinct genetic modifications. Nature 368, 856-859.
Lonberg N, Huszar D (1995) Human antibodies from transgenic mice. Int Rev Immunol. 13, 65-93.
Lowder JN, Meeker TC, Levy R (1985) Monoclonal antibody therapy of lymphoid malignancy. Cancer Surv. 4, 359-375.
McCafferty J, Griffiths AD, Winter G and Chiswell DJ (1990) Phage antibodies: filamentous phage displaying antibody variable domains. Nature 348, 552.
Mellstedt H, Frodin JE, Masucci G, Ragnhammar P, Fagerberg J, Hjelm AL, Shetye J, Wersall P, Osterborg A (1991) The therapeutic use of monoclonal antibodies in colorectal carcinoma. Semin Oncol. 18, 462-477.
Mendez MJ, Green LL, Corvalan JR, Jia XC, Maynard-Currie CE, Yang XD, Gallo ML, Louie DM, Lee DV, Erickson KL, Luna J, Roy CM, Abderrahim H, Kirschenbaum F, Noguchi M, Smith DH, Fukushima A, Hales JF, Klapholz S, Finer MH, Davis CG, Zsebo KM, Jakobovits A (1997) Functional transplant of megabase human immunoglobulin loci recapitulates human antibody response in mice. Nat Genet. 15, 146-156.
Minch SL, Kallio PT, Bailey JE (1995) Tissue plasminogen activator coexpressed in Chinese hamster ovary cells with alpha(2,6)-sialyltransferase contains NeuAc alpha(2,6)Gal beta(1,4)Glc-N-AcR linkages. Biotechn Prog. 11, 348-351.
Morrison SL, Johnson MJ, Herzenberg LA, Oi VT (1984) Chimeric human antibody molecules: mouse antigen-binding domains with human constant region domains. Proc Natl Acad Sci USA. 81,6851-6855.
Muchmore EA, Milewski M, Varki A, Diaz S (1989) Biosynthesis of N-glycolyneuraminic acid. The primary site of hydroxylation of N-acetylneuraminic acid is the cytosolic sugar nucleotide pool. J Biol Chem. 264, 20216-20223.
Nadler L, Stashenko P, Hardy R, Kaplan W, Burton L, Kufe DW, Antman KH, Schlossman SF (1980) Serotherapy of a patient with a monoclonal antibody directed against a human lymphoma-associated antigen. Cancer Res. 40, 3147-3154.
Oi VT, Morrison SL, Herzenberg LA, Berg P (1983) Immunoglobulin gene expression in transformed lymphoid cells. Proc Natl Acad Sci US A. 1983 80, 825-829.
Olive DM, Al-Mulla W, Simsek M, Zarban S, al-Nakib W (1990) The human cytomegalovirus immediate early enhancer-promoter is responsive to activation by the adenovirus-5 13S E1A gene. Arch Virol. 112, 67-80.
Owens RJ and Young RJ. (1994) The genetic engineering of monoclonal antibodies. J. Immunol Methods 168, 149-165.
Reff ME, Carner K, Chambers KS, Chinn PC, Leonard JE, Raab R, Newman RA, Hanna N and Anderson DR (1994) Depletion of B cells in vivo by a chimeric mouse human monoclonal antibody to CD20. Blood 83, 435-445.
Reichmann L, Clark M, Waldmann H and Winter G (1988) Reshaping human antibodies for therapy. Nature 322, 323-327.
Riethmuller G, Riethmuller G, Schneider-Gadicke E, Schlimok G, Schmiegel W, Raab R, Hoffken K, Gruber R, Pichlmaier H, Hirche H, Pichlmayr R, et al. (1994) Randomised trial of monoclonal antibody for adjuvant therapy of resected Dukes' C colorectal carcinoma. Lancet 343, 1177-1183.
Rother RP and Squinto SP (1996) The alpha-Galactosyl epitope: A sugar coating that makes viruses and cells unpalatable. Cell 86, 185-188.
Sanders PG, Wilson RH (1984) Amplification and cloning of the Chinese hamster glutamine synthetase gene. EMBO J. 3, 65-71.
Sandhu JS (1992) Protein Engineering of antibodies. Critical Rev Biotechnology 12, 437-462.
Shawler DL, Bartholomew RM, Smith LM and Dillman RO (1985) Human immune response to multiple injections of murine monoclonal IgG. J Immunol. 135, 1530.
Takeuchi M, Inoue N, Strickland TW, Kubota M, Wada M, Shimizu R, Hoshi S, Kozutsumi H, Takasaki S, Kobata A (1989) Relationship between sugar chain structure and biological activity of recombinant human erythropoietin produced in Chinese hamster ovary cells. Proc Natl Acad Sci USA. 86, 7819-7822.
Urlaub G, Kas E, Carothers AM, Chasin LA (1983) Deletion of the diploid dihydrofolate reductase locus from cultured mammalian cells. Cell 33, 405-412.
Vandamme A-M., Bulens F., Bernar H., Nelles L., Lijnen RH. and Collen D (1990) Construction and characterization of a recombinant murine monoclonal antibody directed against human fibrin fragment-D dimer. Eur J Biochem. 192, 767-775.
Vaswani SK and Hamilton RG (1998) Humanized antibodies as potential therapeutic drugs. Ann Allergy, Asthma and Immunol. 81, 105-115.
Vonach B, Hess B, Leist C (1998) Construction of a novel CHO cell line coexpressing human glucosyltransferases and fusion PSGL-1-immunoglobulin G. In: O.-W.Merten et al (eds), New developments and new applications in animal cell technology, pp.181-183, Kluwer Academic Publishers.
Weikert S, Papac D, Briggs J, Cowfer D, Tom S, Gawlitzek M, Lofgren J, Mehta S, Chisholm V, Modi N, Eppler S, Carroll K, Chamow S, Peers D, Berman P, Krummen L (1999) Engineering Chinese hamster ovary cells to maximize sialic acid content of recombinant glycoproteins. Nature Biotechnology 17, 1116-1121.
White E, Sabbatini P, Debbas M, Wold WS, Kusher DI, Gooding LR (1992) The 19-kilodalton adenovirus E1B transforming protein inhibits programmed cell death and prevents cytolysis by tumor necrosis factor alpha. Mol Cell Biol. 12, 2570-2580.
Winter G, Griffiths AD, Hawkins RE and Hoogenboom HR (1994) Making antibodies by phage display technology. Annu Rev Immunol. 12, 433-455.
Wurm F, Bernard A (1999) Large-scale transient expression in mammalian cells for recombinant protein production. Curr Opin Biotechnol. 10, 156-159.
Yamaguchi K, Akai K, Kawanishi G, Ueda M, Masuda S, Sasaki R (1991) Effects of site-directed removal of N-glycosylation sites in human erythropoietin on its production and biological properties. J Biol Chem. 266, 20434-20439.
Yew PR, Berk AJ (1992) Inhibition of p53 transactivation required for transformation by adenovirus early 1B protein. Nature 357, 82-85.
Zhang X, Lok SH, Kom OL (1998) Stable expression of human alpha-2,6-sialyltransferase in Chinese hamster ovary cells: functional consequences for human erythropoietin expression and bioactivity. Biochim Biophys Acta. 27, 441-452.

### TABLES: Yields of recombinant EPO

**Table 1. Summary of methotrexate (MTX) killing of PER.C6 and PER.C6/E2A after 6 and 15 days of incubation with different MTX concentrations. Cells were seeded at day 0 and incubations with MTX started at day 1 and continued for 6 days. Then confluency (%) was scored and the medium was replaced by fresh medium plus MTX and incubation was continued for another 9 days, after which confluency (%) was scored again (day 15).**

| **PER.C6** | | 0 | 1 | 5 | 10 | 25 | 50 | 100 | 250 | 500 | 1000 | 2500 | nM MTX |
|---|---|---|---|---|---|---|---|---|---|---|---|---|---|
| 1E5 cells/well | day 6 | 70 | 70 | 70 | 60 | <5 | <1 | 0.5 | 0 | 0 | 0 | 0 | % confluency |
| 6-wells plate | day 15 | 100 | 100 | 100 | 100 | <10 | <5 | 0 | 0 | 0 | 0 | 0 | % confluency |
| | | | | | | | | | | | | | |

| **PER.C6/E2A** | | 0 | 1 | 5 | 10 | 25 | 50 | 100 | 250 | 500 | 1000 | 2500 | nM MTX |
|---|---|---|---|---|---|---|---|---|---|---|---|---|---|
| 1E5 cells/well | day 6 | 100 | 100 | 100 | 100 | <100 | 5 | 5 | 4 | 1 | <1 | <1 | % confluency |
| 6-wells plate | day 15 | 100 | 100 | 100 | 100 | <10 | <5 | 0 | 0 | 0 | 0 | 0 | % confluency |

**Table 2. Attached PER.C6 and PER.C6/E2A cell lines that stably express recombinant human EPO. Cell lines were generated by stable integration and expression of pEPO2000/DHFR (figure 5). Production levels were determined in the supernatant, after growth of 4 days in a T25 flask setting in the presence of 100 nM MTX.**

| **PER.C6 cell lines** | ELISA units/1E6 seeded cells/day |
|---|---|
| P3 | 735 |
| P5 | 0 |
| P7 | 1733 |
| P8 | 2522 |
| P9 | 3839 |
| P13 | 0 |
| P15 | 0 |
| P42 | <1 |
| | |

| **PER.C6/E2A cell lines** | ELISA units/1E6 seeded cells/day |
|---|---|
| E17 | 325 |
| E55 | 1600 |

**Table 3. Amplification rate of endogenous and integrated DHFR DNA. The intensities of the hybridizing bands in the Southern blots from figure 19 were measured in a phosphorimager and corrected for background levels to finally calculate the approximate amplification rates of the endogenous and the integrated DHFR genes.**

| **P8** | **E1 probe** | **integrated dhfr** | **amplification** | **endogenous dhfr** | **amplification** |
|---|---|---|---|---|---|
| 100 nM | 719624 | 3375 | | 18649 | |
| 800 nM | 913578 | 2976 | x 0.882 | 45283 | x 2.428 |
| 1800 nM | 831952 | 2950 | x 0.874 | 81506 | x 4.371 |
| | | | | | |

| **P9** | **E1 probe** | **integrated dhfr** | **amplification** | **endogenous dhfr** | **amplification** |
|---|---|---|---|---|---|
| 100 nM | 804142 | 16606 | | 31161 | |
| 1800 nM | 842268 | 14430 | x 0.869 | 69542 | x 2.232 |

**Table 4. EPO yields in transient DNA transfections. Yields per million seeded cells were determined with an EPO ELISA on supernatants from PER.C6, PER.C6/E2A and CHO cells that were transfected with pEPO2000/DHFRwt expression vector in the absence or presence of Fetal Bovine Serum at different incubation temperatures, as described in example 12.**

| **Cell line** | **± FBS** | **Temperature** | **EPO yields (ELISA units/1E6 cells/day)** | | |
|---|---|---|---|---|---|
| PEP-C6/E2A | + | 39C | 3100 | | |
| PER.C6/E2A | - | 39 C | 2600 | | |
| PER.C6 | + | 37 C | 750 | | |
| PER.C6 | - | 37 C | 590 | | |
| CHO | + | 37 C | 190 | | |
| CHO | - | 37 C | 90 | | |

## Claims

1. A PER.C6 cell such as deposited under ECACC no. 96022940, **characterized in that** it further comprises integrated into its genome a cDNA encoding alpha-2,3-sialyltransferase, alpha-2,6-sialyltransferase or beta-1,4-galactosyltransferase.

2. A cell according to claim 1, wherein said cDNA encodes alpha-2,6-sialyltransferase.

3. A cell according to claim 1, wherein said cDNA encodes alpha-2,3-sialyltransferase.

4. A cell according to any one of the preceding claims, said cell further comprising at least one nucleic acid encoding a recombinant protein.

5. A cell according to claim 4, wherein the at least one nucleic acid encoding a recombinant protein is under control of a CMV promoter.

6. A cell according to claim 4 or claim 5, wherein the at least one nucleic acid encoding a recombinant protein is integrated into the genome of said cell.

7. A cell according to any one of claims 4-6, wherein the recombinant protein is a human protein.

8. A cell according to any one of claims 4-7, wherein the recombinant protein is erythropoietin, or a functional derivative, homologue or fragment thereof.

9. A method for producing at least one recombinant protein in a cell, comprising culturing a cell according to any one of claims 4-8 in a suitable medium and harvesting the recombinant protein from said cell and/or said medium.

10. A method according to claim 9, wherein said suitable medium is free of animal-or human-derived serum.

11. A method according to claim 9 or 10, wherein said cell is in suspension during said culturing.

## Patentansprüche

1. PER.C6-Zelle wie unter ECACC-Nr. 96022940 hinterlegt, **dadurch gekennzeichnet, dass** sie ferner eine in ihr Genom integrierte cDNA umfasst, die Alpha-2,3-Sialyltransferase, Alpha-2,6-Sialyltransferase oder Beta-1,4-Galactosyltransferase kodiert.

2. Zelle nach Anspruch 1, wobei die cDNA Alpha-2,6-Sialyltransferase kodiert.

3. Zelle nach Anspruch 1, wobei die cDNA Alpha-2,3-Sialyltransferase kodiert.

4. Zelle nach einem der vorhergehenden Ansprüche, wobei die Zelle ferner mindestens eine Nucleinsäure umfasst, die ein rekombinantes Protein kodiert.

5. Zelle nach Anspruch 4, wobei die mindestens eine Nucleinsäure, die ein rekombinantes Protein kodiert, unter der Kontrolle eines CMV-Promotors ist.

6. Zelle nach Anspruch 4 oder 5, wobei die mindestens eine Nucleinsäure, die ein rekombinantes Protein kodiert, in das Genom der Zelle integriert ist.

7. Zelle nach einem der Ansprüche 4 bis 6, wobei das rekombinante Protein ein menschliches Protein ist.

8. Zelle nach einem der Ansprüche 4 bis 7, wobei das rekombinante Protein Erythropoietin oder ein funktionelles Derivat, Homolog oder Fragment davon ist.

9. Verfahren zur Herstellung von mindestens einem rekombinanten Protein in einer Zelle, umfassend das Züchten einer Zelle nach einem der Ansprüche 4 bis 8 in einem geeigneten Medium und das Ernten des rekombinanten Proteins aus der Zelle und/oder dem Medium.

10. Verfahren nach Anspruch 9, wobei das geeignete Medium frei von aus Tieren oder Menschen stammendem Serum ist.

11. Verfahren nach Anspruch 9 oder 10, wobei die Zelle während des Züchtens in Suspension ist.

## Revendications

1. Cellule PER.C6 telle que déposée sous ECACC n° 96022940, **caractérisée en ce qu'**elle comprend en outre, intégré dans son génome un ADNc codant pour une alpha-2,3-sialyltransférase, une alpha-2,6-sialyltransférase ou une béta-1,4-galactosyltransférase.

2. Cellule selon la revendication 1, dans laquelle ledit ADNc code pour une alpha-2,6-sialyltransférase.

3. Cellule selon la revendication 1, dans laquelle ledit ADNc code pour une alpha-2,3-sialyltransférase.

4. Cellule selon l'une quelconque des revendications précédentes, ladite cellule comprenant en outre au moins un acide nucléique codant pour une protéine recombinante.

5. Cellule selon la revendication 4, dans laquelle le au moins un acide nucléique codant pour une protéine recombinante est sous le contrôle d'un promoteur du CMV.

6. Cellule selon la revendication 4 ou la revendication 5, dans laquelle le au moins un acide nucléique codant pour une protéine recombinante est intégré dans le génome de ladite cellule.

7. Cellule selon l'une quelconque des revendications 4 à 6, dans laquelle la protéine recombinante est une protéine humaine.

8. Cellule selon l'une quelconque des revendications 4 à 7, dans laquelle la protéine recombinante est l'érythropoïétine, ou un dérivé, un homologue ou un fragment fonctionnel de celle-ci.

9. Procédé pour produire au moins une protéine recombinante dans une cellule, comprenant la mise en culture d'une cellule selon l'une quelconque des revendications 4 à 8 dans un milieu approprié et la récolte de la protéine recombinante à partir de ladite cellule et/ou dudit milieu.

10. Procédé selon la revendication 9, dans lequel ledit milieu approprié est exempt de sérum dérivé d'un animal ou d'un humain.

11. Procédé selon la revendication 9 ou 10, dans lequel ladite cellule est en suspension pendant ladite culture.
